# EUROPÄISCHE PATENTSCHRIFT

(11) **EP 3 810 071 B1**
(45) Veröffentlichungstag und Bekanntmachung des Hinweises auf die Patenterteilung: **03.08.2022**
(21) Anmeldenummer: 19731693.8
(22) Anmeldetag: 14.06.2019
(51) Int. Cl.: A61K 8/34, A61K 8/46, A61K 8/81, A61Q 5/08, A61Q 5/10

(54) **OXIDATIVES HAARAUFHELL- ODER HAARFÄRBEMITTEL MIT VERBESSERTEN ANWENDUNGSEIGENSCHAFTEN**
OXIDATIVE HAIR BLEACHING AGENT OR HAIR COLORANT WITH IMPROVED APPLICATION PROPERTIES
AGENT POUR LA COLORATION OU LA DECOLORATION D'OXYDATION DES CHEVEUX AYANT DES PROPRIÉTÉS D'APPLICATION AMÉLIORÉES.

(30) Priorität: 19.06.2018 DE 102018209894
(43) Veröffentlichungstag der Anmeldung: 28.04.2021
(73) Patentinhaber: Henkel AG & Co. KGaA, 40589 Düsseldorf (DE)
(72) Erfinder: SCHOEPGENS, Juergen, 41366 Schwalmtal (DE); ROHLAND, Christa, 40468 Düsseldorf (DE); WESER, Gabriele, 41472 Neuss (DE)
(86) Internationale Anmeldenummer: PCT/EP2019/065697
(87) Internationale Veröffentlichungsnummer: WO 2019/243192

(56) Entgegenhaltungen:
- EP-A1- 3 108 872
- EP-A2- 0 046 543
- EP-A2- 0 640 335
- WO-A1-2018/028861
- DE-A1-102014 226 540

## Beschreibung

Die vorliegende Anmeldung betrifft ein oxidatives Haaraufhell- oder Haarfärbemittel in Cremeform, ein Kit, umfassend dieses Haaraufhell- oder Haarfärbemittel und ein Haaraufhell- oder -färbeverfahren unter Einsatz dieses Haaraufhell- oder Haarfärbemittels.

Für die Aufhellung oder Blondierung von Haaren werden so genannte Oxidationsaufhellmittel verwendet. Für dauerhafte, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, langanhaltende Färbeergebnisse aus. Für natürlich wirkende Färbungen muss aber üblicherweise eine Mischung aus einer größeren Zahl von Oxidationsfarbstoffvorprodukten (OFV) eingesetzt werden. Häufig ist in den Haaraufhell- oder Haarfärbemitteln weiterhin mindestens ein direktziehender Farbstoff (DZ) zur Nuancierung oder zur Mattierung unerwünschter Farbtöne der Melaninabbauprodukte enthalten.

Zumeist weisen oxidative Haaraufhell- und Haarfärbemittel zur Stabilisierung der Farbstoffvorprodukte während der Lagerung und zur Reaktionsbeschleunigung während der oxidativen Anwendung einen alkalischen pH-Wert auf, der mit Alkalisierungsmitteln, wie Alkanolaminen, Ammoniak oder anorganischen Basen, eingestellt wird.

Zur Ausbildung des Farbstoffs wird die alkalische Färbekomponente üblicherweise mit einer wässrigen Wasserstoffperoxidlösung zu einer homogenen Creme oder einem homogenen Gel vermischt und direkt im Anschluss auf die zu färbenden Haare aufgetragen. Zur Aufhellung des Haars durch den oxidativen Abbau des natürlichen Haarfarbstoffs Melanin wird die alkalische Aufhellkomponente üblicherweise mit einer wässrigen Wasserstoffperoxidlösung zu einer homogenen Creme oder einem homogenen Gel vermischt und direkt im Anschluss auf die zu blondierenden Haare aufgetragen. Die Färbe- oder Aufhellmischung verbleibt für eine Zeit von 5 bis 60 Minuten auf dem Haar, bis die oxidative Bildung des Farbstoffs auf dem Haar bzw. der Melaninabbau abgeschlossen sind. Anschließend wird die Färbe- oder Aufhellmischung ausgewaschen.

Die vorgenannten Oxidationsfarbstoffvorprodukte (OFV) und Alkalisierungsmittel sind üblicherweise in einen kosmetisch geeigneten Träger, wie beispielsweise eine Creme, eingearbeitet. Der Träger gewährleistet eine homogene Verteilung und eine ausreichende Verweilzeit des Haaraufhell- oder Haarfärbemittels auf dem Haar. Viele der OFV sind aromatische Aminverbindungen. Einige der gebräuchlichen OFV, wie p-Toluylendiamin oder ,5-Diamino-1-(2-hydroxyethyl)pyrazol, sind nur in Form ihrer Salze, insbesondere als Sulfat, stabil bzw. großtechnisch einsetzbar. Ihr Zusatz erhöht also die Elektrolytkonzentration im Träger. Dies kann zu einer Destabilisierung insbesondere des gel- oder cremeförmigen Trägers führen. Der Träger des erfindungsgemäßen Mittels muss also eine gute Toleranz gegenüber höheren Elektrolytkonzentrationen aufweisen.

Darüber hinaus muss der Träger auch nach der Verdünnung mit der wässrigen Oxidationsmittelzusammensetzung eine anwendungstechnisch geeignete Viskosität aufweisen. Cremeträger mit einem zu hohen Gehalt an hoch schmelzenden Fettkomponenten, wie Fettalkoholen, zeigen häufig einen besonders starken Einbruch der Viskosität der Anwendungsmischung im Vergleich zum oxidationsmittelfreien Träger.

Der vorliegenden Erfindung lag die Aufgabe zu Grunde, ein oxidatives Haaraufhell- oder Haarfärbemittel bereitzustellen, das sich unter möglichst ökonomischen und nachhaltigen Bedingungen herstellen lässt. Weiterhin lag der vorliegenden Erfindung die Aufgabe zu Grunde, ein oxidatives Haaraufhell- oder Haarfärbemittel bereitzustellen, das einfach anzumischen und aufzutragen ist.

Weiterhin lag der vorliegenden Erfindung die Aufgabe zu Grunde, ein oxidatives Haaraufhell- oder Haarfärbemittel bereitzustellen, das ohne höheren Gehalt an hoch schmelzenden Fettkomponenten eine cremeartige Konsistenz, Haptik und Optik aufweist.

Weiterhin lag der vorliegenden Erfindung die Aufgabe zu Grunde, ein oxidatives Haarfärbemittel bereitzustellen, das eine gute Toleranz gegenüber höheren Elektrolytkonzentrationen aufweist. Gelöst werden diese Aufgaben durch ein Mittel zur oxidativen Haaraufhellung oder Haarfärbung, das, jeweils bezogen auf sein Gewicht, Folgendes enthält:
- 70 - 90 Gew.-%, bevorzugt 73 - 86 Gew.-%, besonders bevorzugt 76 - 84 Gew.-%, Wasser,
- mindestens ein Alkalisierungsmittel,
- mindestens ein vernetztes Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, jeweils bezogen auf das Gewicht des Mittels, enthalten ist,
- mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%,
- mindestens ein anionisches Tensid in einer Gesamtmenge von 1 - 6 Gew.-%, bevorzugt 2 - 5 Gew.-%, und besonders bevorzugt 2,5 - 4,5 Gew.-%, und
- mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%,
- optional mindestens eine färbende Substanz, ausgewählt aus Oxidationsfarbstoffvorprodukten, direktziehenden Haarfarbstoffen und Mischungen hiervon,
wobei keine Oxidationsmittel enthalten sind,
dadurch gekennzeichnet, dass
- Natriumpolyacrylat, bevorzugt mit einer massenmittleren Molmasse M_{w} im Bereich von 1.000.000 bis 20.000.000 Dalton, besonders bevorzugt 6.000.000 bis 15.000.000 Dalton, enthalten ist, bevorzugt in einer Gesamtmenge von 0,1 - 1,5 Gew.-%, bevorzugt 0,5 - 1,3 Gew.-%, besonders bevorzugt 0,8 - 1,1 Gew.-%, jeweils bezogen auf das Gewicht des Mittels, wobei besonders bevorzugt das Natriumpolyacrylat als in einer Wasser-in-ÖI-Emulsion vorgeliert enthalten ist.

Das erfindungsgemäße Mittel stellt die alkalische Färbekomponente eines oxidativen Haaraufhell- oder Haarfärbemittels dar. Diese wird üblicherweise unmittelbar vor der Anwendung mit einer wässrigen Wasserstoffperoxid-Zubereitung vermischt und dann auf das aufzuhellende bzw. zu färbende Haar appliziert. Bis zum Vermischen mit der wässrigen Wasserstoffperoxid-Zubereitung enthält das erfindungsgemäße Mittel keine Oxidationsmittel.

### Wassergehalt

Das erfindungsgemäße Mittel enthält, jeweils bezogen auf sein Gewicht, 70 - 90 Gew.-%, bevorzugt 73 - 86 Gew.-%, besonders bevorzugt 76 - 84 Gew.-%, Wasser.

### Alkalisierungsmittel

Das erfindungsgemäße Mittel enthält mindestens ein Alkalisierungsmittel. Das zur Einstellung des bevorzugten pH-Wertes erfindungsgemäß bevorzugte Alkalisierungsmittel ist ausgewählt aus der Gruppe, umfassend Ammoniumhydroxid, basische Aminosäuren, Alkalihydroxide, Alkanolamine, Alkalimetallmetasilikate, Alkaliphosphate und Alkalihydrogenphosphate sowie Mischungen hiervon. Als Alkalimetallionen dienen bevorzugt Lithium, Natrium, Kalium, insbesondere Natrium oder Kalium.

Die als Alkalisierungsmittel einsetzbaren basischen Aminosäuren sind bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide sind bevorzugt ausgewählt aus Natriumhydroxid und Kaliumhydroxid.

Die als Alkalisierungsmittel einsetzbaren Alkanolamine sind bevorzugt ausgewählt aus primären Aminen mit einem C₂-C₆-Alkylgrundkörper, der mindestens eine Hydroxylgruppe trägt. Besonders bevorzugte Alkanolamine sind aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopro-pan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine sind ausgewählt aus der Gruppe 2-Aminoethan-1-ol, 2-Amino-2-methylpropan-1-ol und 2-Amino-2-methyl-propan-1,3-diol.

Ein erfindungsgemäß besonders bevorzugtes Alkalisierungsmittel ist Monoethanolamin (2-Amino-ethan-1-ol). Zur Erzielung eines möglichst geruchsfreien Färbeverfahrens und zur Optimierung der Echtheitseigenschaften der Färbung ist Monoethanolamin in einer Gesamtmenge von 0,2 - 10 Gew.-%, bevorzugt von 0,5 - 8 Gew.-%, weiter bevorzugt von 1 bis 6 Gew.-% und besonders bevorzugt von 2 bis 4 Gew.-% - bezogen auf das Gewicht des erfindungsgemäßen Aufhell- oder Färbemittels - enthalten.

Zusätzlich zu bzw. anstelle von Monoethanolamin enthalten weitere bevorzugte erfindungsgemäße Aufhell- und Färbemittel Ammoniumhydroxid, also Ammoniak in Form seiner wässrigen Lösung. Bei entsprechenden wässrigen Ammoniak-Lösungen kann es sich um 10 bis 35 prozentige Lösungen handeln (berechnet in Vol.-%. 100 g wässrige Ammoniaklösung mit 25 Vol.-% NH₃ enthalten etwa 50 g Ammoniak. Bevorzugt wird Ammoniak in Form einer 20 bis 30 Vol.-%igen Lösung, besonders bevorzugt in Form einer 25 Vol.-%igen Lösung eingesetzt.

In einer besonders bevorzugten Ausführungsform enthält das erfindungsgemäße Aufhell- oder Färbemittel Ammoniumhydroxid in einer Menge von 0,2 bis 6 Gew.-%, bevorzugt von 0,3 bis 5 Gew.-%, weiter bevorzugt von 0,5 bis 3 Gew.-% und besonders bevorzugt von 1 bis 2 Gew.-%, bezogen auf das Gewicht des erfindungsgemäßen Aufhell- oder Färbemittels.

Weiterhin können andere Alkalisierungsmittel, wie Kaliumhydroxid und Natriumhydroxid enthalten sein, bevorzugt in einer Gesamtmenge von 0,05 bis 1,5 Gew.-%, besonders bevorzugt 0,1 bis 0,6 Gew.-%, jeweils bezogen auf das Gewicht des erfindungsgemäßen Aufhell- oder Färbemittels.

In einer weiteren besonders bevorzugten Ausführungsform enthält das erfindungsgemäße Aufhell- oder Färbemittel mindestens ein Alkalisierungsmittel, das ausgewählt ist aus der Gruppe, umfassend Alkanolamine, Kaliumhydroxid, Natriumhydroxid sowie Mischungen hiervon.

In einer weiteren besonders bevorzugten Ausführungsform enthält das erfindungsgemäße Aufhell- oder Färbemittel mindestens ein Alkalisierungsmittel in einer Gesamtmenge von 0,02 - 0,4 mol/100 g, bevorzugt 0,05 - 0,3 mol/100g, jeweils in mol Alkalisierungsmittel pro 100 Gramm des erfindungsgemäßen Mittels.

Bevorzugte erfindungsgemäße Mittel sind gekennzeichnet durch einen pH-Wert im Bereich von 7,5 - 12, bevorzugt 9 - 11, besonders bevorzugt 9,5 - 10,5, jeweils gemessen bei 20°C.

Ein weiteres wesentliches Merkmal der erfindungsgemäßen Mittel ist der Gehalt an mindestens einem vernetzten Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, jeweils bezogen auf das Gewicht des Mittels, enthalten ist. Bevorzugt ist das mindestens eine vernetzte Copolymer aus Acrylsäure und nicht-ethoxylierten Estern der Acrylsäure mit linearen C10-C30-Monoalkoholen ausgewählt aus Copolymeren mit der INCI-Bezeichnung Acrylates/C10-30 Alkyl Acrylate Crosspolymer. Als Vernetzungsagenz ist bevorzugt Sucroseallylether oder Pentaerythritylallylether enthalten.

Erfindungsgemäß besonders bevorzugte vernetzte Copolymere aus Acrylsäure und nicht-ethoxylierten Estern der Acrylsäure mit linearen C10-C30-Monoalkoholen sind erhältlich durch Polymerisation einer Monomermischung, die - jeweils bezogen auf ihr Gewicht - 80 bis 99 Gew.-%, bevorzugt 90 bis 98 Gew.-%, Acrylsäure, mindestens einen nicht-ethoxylierten Ester der Acrylsäure mit linearen C10-C30-Monoalkoholen in einer Gesamtmenge von 0,9 - 19,9 Gew.-%, bevorzugt 2 - 10 Gew.-% sowie mindestens ein Vernetzungsagenz in einer Gesamtmenge von 0,1 - 4 Gew.-% enthält.

Weitere erfindungsgemäß besonders bevorzugte vernetzte Copolymere aus Acrylsäure und nicht-ethoxylierten Estern der Acrylsäure mit linearen C10-C30-Monoalkoholen sind dadurch gekennzeichnet, dass ihre 0,5 Gew.-%ige Dispersion in Wasser bei 25 °C und einem pH-Wert im Bereich von 5,8 - 6,3 eine Viskosität im Bereich von 45.000 bis 65.000 mPas aufweist, gemessen mit einem Brookfield RVF- oder Brookfield RVT-Viskosimeter bei einer Rotationsfrequenz von 20 min⁻¹ mit Spindel #7.

Der Gehalt an dem mindestens einen vernetzten Copolymer, aufgebaut aus Acrylsäure und nicht-ethoxylierten Estern der Acrylsäure mit linearen C10-C30-Monoalkoholen als Monomeren, ist so gewählt, dass die Viskosität des erfindungsgemäßen Mittels bevorzugt im Bereich von 8000 - 40.000 mPas, bevorzugt 10.000 - 35.000 mPas, besonders bevorzugt 12.000 - 30.000 mPas, liegt, jeweils gemessen bei 20°C mit einem Haake VT 550 Viskosimeter mit dem Messsystem SV.

Das Messprogramm des Haake VT 550 Viskosimeters lautet sowohl für das Messsystem SV als auch für das Messsystem MV II wie folgt (s = Sekunde):
- Rotationsrampe von 0/s bis 57,6/s Scherung im Zeitraum von 48 s
- während der Messzeit: Datenaufnahme von 100 Datenpunkten
- Interpolation der Werte über: y = a + bx + d
- Erfassung der dynamischen Viskosität bei 7,2/s Scherung.

Die Messsysteme SV und MV II unterscheiden sich durch die Spindel und die Größe des Messbechers voneinander.

Die erfindungsgemäßen und erfindungsgemäß verwendeten Mittel enthalten weiterhin mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%, jeweils bezogen auf das Gewicht des Mittels. Erfindungsgemäß bevorzugt ist das mindestens eine lineare, gesättigte 1-Alkanol mit einer Hydroxygruppe und 8 bis 22 Kohlenstoffatomen ausgewählt aus 1-Decanol, 1-Dodecanol (Laurylalkohol), 1-Tridecanol, 1-Tetradecanol (Myristylalkohol), 1-Hexadecanol (Cetylalkohol), 1-Octadecanol (Stearylalkohol), 1-Eicosanol (Arachylalkohol) und 1-Docosanol (Behenylalkohol) sowie Mischungen hiervon. Eine erfindungsgemäß bevorzugte Alkanolmischung ist Kokosalkohol, also Alkanolmischungen, die durch Hydrierung von Kokosöl erhalten werden. Ein erfindungsgemäß besonders bevorzugter Kokosalkohol weist, jeweils bezogen auf sein Gewicht, folgende Kettenlängen-Verteilung auf: C₁₀ und kürzer: null bis maximal 3 Gew.-%, C₁₂: 48 - 58 Gew.-%, C₁₄: 18-24 Gew.-%, C₁₆: 8-12 Gew.-%, C₁₈: 11-15 Gew.-%, C₂₀: null bis maximal 1 Gew.-%. Erfindungsgemäß außerordentlich bevorzugte C₈-C₂₂-Alkan-1-ole sind ausgewählt aus Laurylalkohol, Kokosalkohol,1-Tetradecanol (Myristylalkohol), Cetylalkohol und Stearylalkohol sowie Mischungen hiervon, insbesondere aus Kokosalkohol und Laurylalkohol.

Im Sinne der vorliegenden Anmeldung werden die vorgenannten linearen, gesättigten 1-Alkanole mit einer Hydroxygruppe sowohl in Bezug auf die erfindungsgemäßen Mittel und Zusammensetzungen (M1) als auch in Bezug auf die Oxidationsmittelzubereitungen (M2) nicht zu den Tensiden gezählt.

### Aniontensid

Die erfindungsgemäßen und erfindungsgemäß verwendeten Mittel enthalten mindestens ein anionisches Tensid in einer Gesamtmenge von 1 - 6 Gew.-%, bevorzugt 2 - 5 Gew.-%, und besonders bevorzugt 2,5 - 4,5 Gew.-%, jeweils bezogen auf das Gewicht des Mittels.

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen.

Im Sinne der vorliegenden Anmeldung werden gesättigte und ungesättigte Alkan-1-ole mit mindestens 4 Kohlenstoffatomen im Alk(en)ylrest und Glycerylfettsäuremono- und -diester mit mindestens 4 Kohlenstoffatomen im Fettsäurerest nicht zu den Tensiden gezählt.

Der hydrophobe Rest ist bevorzugt eine Kohlenwasserstoffkette mit 8-30 Kohlenstoff-Atomen, die gesättigt oder ungesättigt, linear oder verzweigt sein kann. Besonders bevorzugt ist diese C₈-C₃₀-Alkylkette linear. Basiseigenschaften der Tenside und Emulgatoren sind die orientierte Adsorption an Grenzflächen sowie die Aggregation zu Mizellen und die Ausbildung von lyotropen Phasen.

Bei der Auswahl erfindungsgemäß geeigneter Tenside kann es bevorzugt sein, ein Gemisch von Tensiden einzusetzen, um die Eigenschaften der erfindungsgemäßen Oxidationsaufhell- oder -färbemittel optimal einzustellen.

Als anionische Tenside eignen sich für die erfindungsgemäßen Mittel alle für die Verwendung am menschlichen Körper geeigneten anionischen oberflächenaktiven Stoffe, die eine wasserlöslich machende, anionische Gruppe, beispielsweise eine Sulfat-, Sulfonat- oder Phosphat-Gruppe, und eine lipophile Alkylgruppe mit etwa 8 bis 30 C-Atomen, bevorzugt 8 bis 24 C-Atomen im Molekül aufweisen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe, polyethoxylierte Ethercarbonsäuren, Acylsarcoside, Acyltauride, Acylisethionate, Sulfobernsteinsäuremono- und -dialkylester und Sulfobernsteinsäuremono-alkylpolyoxyethylester mit 1 bis 6 Ethylenoxidgruppen, lineare Alkansulfonate, lineare alpha-Olefinsulfonate, Sulfonate ungesättigter Fettsäuren mit bis zu 6 Doppelbindungen, alpha-Sulfofettsäuremethylester von Fettsäuren, C₈-C₂₀-Alkylsulfate und C₈-C₂₀-Alkylethersulfate mit 1 bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, lineare und verzweigte Fettsäuren mit 8 bis 30 C-Atomen und deren Salze (Seifen), sowie Monoglyceridsulfate und Monoglyceridethersulfate. Bevorzugte anionische Tenside sind ausgewählt aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül. Besonders bevorzugt ist Natriumlaurethsulfat mit 1 bis 3, besonders bevorzugt 2, Ethylenoxidgruppen im Molekül.

Erfindungsgemäß außerordentlich bevorzugte Mittel enthalten mindestens ein anionisches Tensid, ausgewählt aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei Natriumlaurethsulfat mit 1 bis 3, besonders bevorzugt 2, Ethylenoxidgruppen im Molekül, in einer Gesamtmenge von 1 - 6 Gew.-%, bevorzugt 2 - 5 Gew.-%, und besonders bevorzugt 2,5 - 4,5 Gew.-%, jeweils bezogen auf das Gewicht des Mittels, besonders bevorzugt ist.

### Niotensid

In einer bevorzugten Ausführungsform der Erfindung enthalten erfindungsgemäße und erfindungsgemäß verwendete Mittel mindestens ein nichtionisches Tensid. Besonders bevorzugt ist mindestens ein nichtionisches Tensid in einer Gesamtmenge von 0,01 - 1 Gew.-%, bevorzugt 0,05 - 0,5 Gew.-%, und besonders bevorzugt 0,1 - 0,3 Gew.-%, jeweils bezogen auf das Gewicht des Mittels, enthalten.

Als nichtionische Tenside eignen sich für die erfindungsgemäßen Mittel alle für die Verwendung am menschlichen Körper geeigneten nichtionischen oberflächenaktiven Stoffe, die mindestens eine wasserlöslich machende, nichtionische Gruppe aufweisen, insbesondere eine Polyethylenglykolether-Gruppe mit mindestens 2 Ethylenoxid-Einheiten, eine Glycosid-Gruppe, insbesondere eine Glucose- oder Methylglucose-Gruppe, eine Polyglycosid-Gruppe mit im Mittel mehr als einer Glycosid-Einheit, eine Polyglycerin-Gruppe mit mindestens zwei Glycerineinheiten, eine Sorbitan-Gruppe, eine Amidgruppe oder mehrere verschiedene dieser Gruppen, beispielsweise eine Sorbitan-Gruppe und eine Polyethylenglykolether-Gruppe, und eine lipophile Alkylgruppe mit etwa 8 bis 30 C-Atomen, bevorzugt 10 bis 24 C-Atomen. Besonders bevorzugt verwendete nichtionische Tenside sind ausgewählt sind aus mit 7 - 80 Mol Ethylenoxid pro Mol ethoxyliertem Rizinusöl, ethoxylierten C₈-C₃₀-Alkanolen mit 4-100 Mol Ethylenoxid pro Mol, ethoxylierten C₈-C₃₀-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen.

Die ethoxylierten C₈-C₃₀-Alkanole haben die Formel R¹O(CH₂CH₂O)ₙH, wobei R¹ steht für einen linearen oder verzweigten Alkyl- und/oder Alkenylrest mit 8 - 30 Kohlenstoffatomen und n, die mittlere Anzahl der Ethylenoxid-Einheiten pro Molekül, für Zahlen von 4 - 100, bevorzugt 6 - 30, besonders bevorzugt 12 bis 20 Mol Ethylenoxid an 1 Mol Alkanol, das bevorzugt ausgewählt ist aus Caprylalkohol, 2-Ethylhexylalkohol, Caprinalkohol, Laurylalkohol, Isotridecylalkohol, Tridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie aus deren technischen Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Trideceth-6, Isotrideceth-6, Undeceth-6, Myreth-6, Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12, Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Coceth-30; außerordentlich bevorzugt sind Ceteth-10, Ceteth-12, Ceteth-15, Ceteth-20, Ceteth-30, Steareth-10, Steareth-12, Steareth-15, Steareth-20 und Steareth-30 sowie Mischungen hiervon. Die ethoxylierten C₈-C₃₀-Carbonsäuren haben die Formel R¹O(CH₂CH₂O)ₙH, wobei R¹O steht für einen linearen oder verzweigten gesättigten oder ungesättigten Acylrest mit 8 -30 Kohlenstoffatomen und n, die mittlere Anzahl der Ethylenoxid-Einheiten pro Molekül, für Zahlen von 5- 30, bevorzugt 6 - 20, besonders bevorzugt 6 bis 12 Mol Ethylenoxid an 1 Mol C₈-C₃₀-Carbonsäure, die bevorzugt ausgewählt ist aus Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie aus deren technischen Mischungen. Auch Addukte von 5 - 30, bevorzugt 6 - 20, besonders bevorzugt 6 bis 12 Mol Ethylenoxid an technische Fettsäuren mit 12 - 18 Kohlenstoffatomen, wie Kokos-, Palm-, Palmkern- oder Talgfettsäure, sind geeignet.

Alkylmono- und -oligoglycoside mit 8 bis 22 Kohlenstoffatomen im Alkylrest stellen bekannte nichtionische Tenside gemäß Formel (I) dar,

R¹O-[G]ₚ (I)

in der R¹ für einen Alkyl- oder Alkenylrest mit 8 bis 22 Kohlenstoffatomen, G für einen Zuckerrest mit 5 oder 6 Kohlenstoffatomen und p für Zahlen von 1 bis 10 steht. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/ oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Bevorzugt sind solche Alkyl- und/oder Alkenyloligoglykoside, deren Oligomerisierungsgrad kleiner als 1,7 ist und insbesondere im Bereich von 1,2 bis 1,4 liegt. Der Alkyl- bzw. Alkenylrest R¹ kann sich von primären Alkoholen mit 4 bis 22, vorzugsweise 8 bis 22 Kohlenstoffatomen ableiten. Typische Beispiele sind Caprylalkohol, Caprinalkohol, Undecylalkohol, Laurylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol, natürliche Fettalkohole wie Kokosalkohol sowie technische Mischungen. Beispiele für im Handel erhältliche Alkyloligoglucosid-Produkte sind die Oramix^{®} - Typen der Fa. Seppic, beispielsweise Oramix^{®} NS 10, und die Plantacare^{®} - Typen von BASF, beispielsweise Plantacare^{®} 2000UP, Plantacare^{®} 1200UP, Plantacare^{®} 810UP und Plantacare^{®} 818UP. Weiterhin besonders bevorzugt ist Cocoglucosid, ein nichtionisches Tensid gemäß der vorstehenden Formel (I), in der R¹ für Kokosalkylreste mit 8 bis 16 Kohlenstoffatomen, G für einen Glucoserest und p für Zahlen im Bereich von 1,2 bis 1,4 steht.

Erfindungsgemäß außerordentlich bevorzugte Mittel enthalten mindestens ein nichtionisches Tensid, ausgewählt aus mit 7 - 80 Mol Ethylenoxid pro Mol ethoxyliertem Rizinusöl, ethoxylierten C₈-C₃₀-Alkanolen mit 6 - 30, bevorzugt 12 bis 20 Mol Ethylenoxid pro Mol, ethoxylierten C₈-C₃₀-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂ - C₃₀-Carbonsäuren, die hydroxyliert sein können, Alkylmono- und -oligoglycosiden mit 8 bis 22 Kohlenstoffatomen im Alkylrest, sowie Mischungen der vorgenannten Substanzen, in einer Gesamtmenge von 0,01 - 1 Gew.-%, bevorzugt 0,05 - 0,5 Gew.-%, und besonders bevorzugt 0,1 - 0,3 Gew.-%, jeweils bezogen auf das Gewicht des Mittels.

Weitere erfindungsgemäß außerordentlich bevorzugte Mittel enthalten eine Mischung aus Natriumlaurethsulfat mit 1 bis 3, besonders bevorzugt 2, Ethylenoxidgruppen im Molekül und mindestens einem nichtionischen Tensid, ausgewählt aus ethoxylierten C₈-C₃₀-Alkanolen mit 6 - 30, bevorzugt 12 bis 20 Mol Ethylenoxid pro Mol, und Alkylmono- und -oligoglycosiden mit 8 bis 22 Kohlenstoff-atomen im Alkylrest in einer Gesamtmenge von 1,01 - 7 Gew.-%, bevorzugt 2,05 - 5,5 Gew.-%, und besonders bevorzugt 2,6 - 4,8 Gew.-%, jeweils bezogen auf das Gewicht des Mittels.

Erfindungsgemäße und erfindungsgemäß verwendete Mittel enthalten mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%, jeweils bezogen auf das Gewicht des Mittels.

Überraschenderweise wurde gefunden, dass der Zusatz eines kationischen und zwitterionischen Polymers in einer Gesamtmenge von 0,1 - 2 Gew.-% eine weitere Verbesserung der Cremegel-Struktur bewirkt. Außerdem führt das kationische oder zwitterionische Polymer dazu, dass auch nach dem Vermischen mit einer wässrigen Oxidationsmittelzusammensetzung eine anwendungstechnisch optimale Viskosität erhalten wird.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, die aus gleichen, sich wiederholenden organischen Einheiten bestehen. Polymere werden durch Polymerisation von einem Monomertyp oder durch Polymerisation von verschiedenen, strukturell voneinander unterschiedlichen Monomertypen hergestellt. Wird das Polymer durch Polymerisation eines Monomertyps hergestellt, spricht man von Homopolymeren. Werden strukturell unterschiedliche Monomertypen in der Polymerisation eingesetzt, spricht der Fachmann von Copolymeren.

Das maximale Molekulargewicht des Polymers hängt vom Polymerisationsgrad (Anzahl der polymerisierten Monomere) ab und wird durch die Polymerisationsmethode mitbestimmt. Im Sinne der vorliegenden Erfindung ist es bevorzugt, wenn das Molekulargewicht des zwitterionischen Polymers 100.000 bis 10⁷ g/mol, bevorzugt 200.000 bis 5·10⁶ g/mol und besonders bevorzugt 500.000 bis 1·10⁶ g/mol beträgt.

Unter zwitterionischen Polymeren werden solche Polymere verstanden, die im Makromolekül sowohl kationische Gruppierungen als auch anionische Gruppierungen enthalten. Bei den im Makromolekül enthaltenen kationischen Gruppierungen handelt es sich quartäre Ammoniumgruppen. Bei diesen quartären Ammoniumgruppen trägt ein positiv geladenes Stickstoffatom vier organische Reste. Bei den anionischen Gruppierungen handelt es sich um -COO⁻-Gruppen oder -SO₃⁻-Gruppen.

Die kationischen Polymere können Homo- oder Copolymere oder Polymere auf Basis natürlicher Polymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen, wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Bevorzugte kationische und zwitterionische Polymere, die sich als besonders wirkungsvolle Bestandteile der erfindungsgemäßen Wirkstoffkombination erwiesen haben, sind ausgewählt aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminium-salzen und Acrylsäure,
- Copolymere aus N,N,N-Trimethyl-3-[(2-methyl-1-oxo-2-propen-1-yl)amino]-1-propanaminium-salzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminium-salzen, Acrylsäure und Acrylamid, z.B. Polyquaternium-53
- Copolymere aus N,N,N-Trimethyl-3-[(2-methyl-1-oxo-2-propen-1-yl)amino]-1-propanaminium-salzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z. B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure,
- Copolymere, die mindestens eine anionische Struktureinheit der Formel (II) und mindestens eine kationische Struktureinheit der Formel (III) enthalten 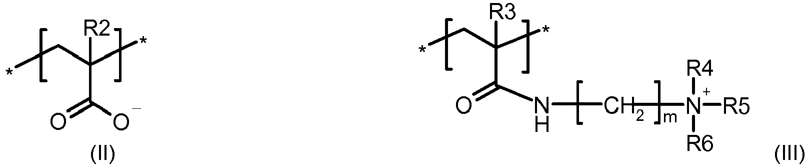 worin R2 und R3 unabhängig voneinander für ein Wasserstoffatom oder eine Methylgruppe stehen, m für eine ganze Zahl von 2 bis 6, bevorzugt für die Zahlen 2 oder 3, steht und die Reste R4, R5 und R6 unabhängig voneinander für eine C₁-C₆-Alkylgruppe, bevorzugt unabhängig voneinander für eine Methylgruppe, eine Ethylgruppe oder eine Propylgruppe, stehen, wobei ein besonders bevorzugtes zwitterionisches Polymer dieses Typs, das gemäß DE3929973A1, Herstellungsbeispiel 1 hergestellt wurde, unter dem INCI-Namen Acrylamidopropyltrimonium chloride / Acrylate Copolymer bekannt ist;
- polymere Dimethyldiallylammoniumsalze und deren Copolymere mit Estern und Amiden von Acrylsäure und Methacrylsäure. Besonders bevorzugte Polymere dieses Typs sind Dimethyldiallylammoniumchlorid-Acrylamid-Copolymere, insbesondere solche mit der INCI-Bezeichnung Polyquaternium-7. Polyquaternium-7 ist zum Beispiel als Handelsprodukt Merquat^{®}550 erhältlich. Ein anderes bevorzugtes Polymer dieses Typs ist das Homopolymer Poly(dimethyldiallylammoniumchlorid), insbesondere die Homopolymere mit der INCI-Bezeichnung Polyquaternium-6. Polyquaternium-6 ist zum Beispiel als Handelsprodukt Merquat^{®}100 erhältlich. Weitere bevorzugte Polymere dieses Typs sind Terpolymere aus Dimethyldiallylammoniumchlorid, Acrylamid und Ammoniumacrylat, insbesondere solche mit der INCI-Bezeichnung Polyquaternium-39. Polyquaternium-39 ist zum Beispiel als Handelsprodukt Merquat^{®}3330 und Merquat^{®}3331 erhältlich. Weitere bevorzugte Polymere dieses Typs sind Copolymere aus Dimethyldiallylammoniumchlorid und Acrylsäure, insbesondere solche mit der INCI-Bezeichnung Polyquaternium-22. Polyquaternium-22 ist zum Beispiel als Handelsprodukt Merquat^{@}280 erhältlich;
- Homopolymere der allgemeinen Formel -{CH₂-[CR¹COO-(CH₂)ₘN⁺R²R³R⁴]}ₙ X⁻, in der R¹= -H oder -CH₃ ist, R², R³ und R⁴ unabhängig voneinander ausgewählt sind aus C1-4-Alkyl-, C1-4-Alkenyl- oder C1-4-Hydroxyalkylgruppen, m = 1, 2, 3 oder 4, n eine natürliche Zahl und X⁻ ein physiologisch verträgliches organisches oder anorganisches Anion ist. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2.Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfate und Halogenidionen, insbesondere Chlorid.

Weitere bevorzugt geeignete kationische Polymere, die von synthetischen Polymeren abgeleitet sind, sind beispielsweise Copolymere aus A1) 0,1 bis 50 %, vorzugsweise 10 bis 50 % (bezogen auf die Gesamt-Anzahl an Monomeren im Copolymer) Monomeren der Formel (IV) 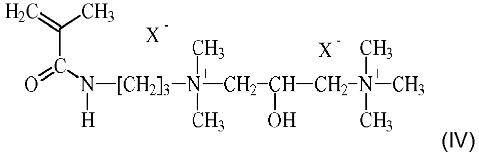 in der X steht für Chlorid, Sulfat, Methosulfat, und
A2) Monomere aus der Gruppe Acrylsäure, Methacrylsäure sowie den Alkalimetall- und Ammoniumsalzen dieser Säuren, wobei das Monomere A2 50 bis 99,9 %, vorzugsweise 50 bis 90 % (bezogen auf die Gesamt-Anzahl an Monomeren im Copolymer) des Copolymers ausmacht; enthalten.

Ein höchst bevorzugtes Polymer, das wie zuvor dargestellt aufgebaut ist, ist unter der INCI-Bezeichnung Polyquaternium-74 im Handel erhältlich.

Ein besonders geeignetes Homopolymer ist das, gewünschtenfalls vernetzte, Poly(methacryloyl-oxyethyltrimethylammoniumchlorid) mit der INCI-Bezeichnung Polyquaternium-37, - sowie Mischungen hiervon.

Weitere erfindungsgemäß bevorzugte oder bevorzugt verwendete Mittel sind dadurch gekennzeichnet, dass mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%, jeweils bezogen auf das Gewicht des Mittels, enthalten ist.

Weitere erfindungsgemäß besonders bevorzugte oder besonders bevorzugt verwendete Mittel sind dadurch gekennzeichnet, dass mindestens ein kationisches oder zwitterionisches Polymer, ausgewählt aus Polyquaternium-22, Polyquaternium-39, Acrylamidopropyltrimonium chloride/Acrylate Copolymer und Polyquaternium-6 sowie Mischungen hiervon, wobei Polyquaternium-22 außerordentlich bevorzugt ist, bevorzugt in einer Gesamtmenge von 0,1 - 2 Gew.-%, besonders bevorzugt 0,2 - 1,5 Gew.-%, außerordentlich bevorzugt 0,35 - 0,5 Gew.-%, jeweils bezogen auf das Gewicht des Mittels, enthalten ist.

Weiterhin enthalten die erfindungsgemäßen Mittel Natriumpolyacrylat. Unter Natriumpolyacrylat werden erfindungsgemäß bevorzugt Polymere mit der CAS-Nummer 9003-04-7 verstanden. Erfindungsgemäß bevorzugte Natriumpolyacrylate weisen eine massenmittlere Molmasse M_{w} im Bereich von 1.000.000 bis 20.000.000 Dalton, bevorzugt 6.000.000 bis 15.000.000 Dalton auf. Das mittlere Molekulargewicht M_{w} kann beispielsweise durch Gelpermeationschromatographie (GPC) mit Polystyrol als internem Standard gemäß DIN 55672-3, Version 8/2007, bestimmt werden.

Die erfindungsgemäße Mischung aus vernetztem Copolymer aus Acrylsäure und nicht-ethoxylierten Estern der Acrylsäure mit linearen C10-C30-Monoalkoholen, linearem, gesättigtem C8-C22-Alkan-1-ol, Aniontensid und Natriumpolyacrylat führt zu einer Verdickung des Mittels mit anwendungstechnisch optimaler Viskosität, wobei das Mittel gleichzeitig die Konsistenz einer Gel-ähnlichen Creme mit einer hervorragenden Haptik erhält.

Erfindungsgemäß außerordentlich bevorzugte Mittel enthalten Natriumpolyacrylat in einer Gesamtmenge von 0,1 - 1,5 Gew.-%, bevorzugt 0,5 - 1,3 Gew.-%, besonders bevorzugt 0,8 - 1,1 Gew.-%, jeweils bezogen auf das Gewicht des Mittels.

In einer besonders bevorzugten Ausführungsform ist das Natriumpolyacrylat als in einer Wasser-in-Öl-Emulsion vorgeliertes Natriumpolyacrylat enthalten. Hierbei ist besonders bevorzugt, dass die Natriumpolyacrylat-haltige Wasser-in-ÖI-Emulsion, jeweils bezogen auf ihr Gewicht, 40 - 60 Gew.-% Natriumpolyacrylat, insgesamt 25 - 45 Gew.-% Öl(e), insgesamt 0,5 - 4,9 Gew.-% Tensid(e) und 0,5 - 4,9 Gew.-% Wasser enthält.

Besonders bevorzugt ist das in der Natriumpolyacrylat-haltigen Wasser-in-ÖI-Emulsion enthaltene Öl ausgewählt aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen; den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen. Ein erfindungsgemäß besonders bevorzugtes Öl ist Mineralöl.

Besonders bevorzugt ist das mindestens eine in der Natriumpolyacrylat-haltigen Wasser-in-ÖI-Emulsion enthaltene Tensid ausgewählt aus nichtionischen Tensiden. Besonders bevorzugt verwendete nichtionische Tenside sind ausgewählt sind aus mit 7 - 80 Mol Ethylenoxid pro Mol ethoxyliertem Rizinusöl, ethoxylierten C₈-C₂₄-Alkanolen mit 5 - 30 Mol Ethylenoxid pro Mol, ethoxylierten C₈-C₂₄-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 30, bevorzugt 6 - 20, besonders bevorzugt 6 bis 12 Mol Ethylenoxid an 1 Mol Alkanol, das bevorzugt ausgewählt ist aus Caprylalkohol, 2-Ethylhexylalkohol, Caprinalkohol, Laurylalkohol, Isotridecylalkohol, Tridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie aus deren technischen Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Trideceth-6, Isotrideceth-6, Undeceth-6, Myreth-6, Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12, Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Coceth-30; außerordentlich bevorzugt sind Trideceth-6 und Isotrideceth-6 sowie Mischungen hiervon.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5- 30, bevorzugt 6 - 20, besonders bevorzugt 6 bis 12 Mol Ethylenoxid an 1 Mol C₈-C₂₄-Carbonsäure, die bevorzugt ausgewählt ist aus Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie aus deren technischen Mischungen. Auch Addukte von 5 - 30, bevorzugt 6 - 20, besonders bevorzugt 6 bis 12 Mol Ethylenoxid an technische Fettsäuren mit 12 - 18 Kohlenstoff-atomen, wie Kokos-, Palm-, Palmkern- oder Talgfettsäure, sind geeignet.

Erfindungsgemäß außerordentlich bevorzugte Mittel sind dadurch gekennzeichnet, dass sie mindestens ein Natriumpolyacrylat mit einer massenmittleren Molmasse M_{w} im Bereich von 1.000.000 bis 20.000.000 Dalton, bevorzugt 6.000.000 bis 15.000.000 Dalton, in einer Gesamtmenge von 0,1 - 1,5 Gew.-%, bevorzugt 0,5 - 1,3 Gew.-%, besonders bevorzugt 0,8 - 1,1 Gew.-%, jeweils bezogen auf das Gewicht des Mittels, enthalten, wobei das Natriumpolyacrylat als in einer Wasser-in-ÖI-Emulsion vorgeliert enthalten ist, wobei besagte Wasser-in-ÖI-Emulsion, jeweils bezogen auf ihr Gewicht, 40 - 60 Gew.-% Natriumpolyacrylat, insgesamt 25 - 45 Gew.-% Öl(e), bevorzugt Mineralöl, insgesamt 0,5 - 4,9 Gew.-% Tensid(e), bevorzugt 0,5 - 4,9 Gew.-% Niotensid(e), und 0,5 - 4,9 Gew.-% Wasser enthält.

Die Kombination aus dem vernetzten Copolymer aus Acrylsäure und nicht-ethoxylierten Estern der Acrylsäure mit linearen C10-C30-Monoalkoholen, linearem, gesättigten 1-Alkanol, Aniontensid, Kation- oder Zwitterionpolymer und Natriumpolyacrylat bewirkt eine besonders reichhaltige, cremige Konsistenz und Haptik der erfindungsgemäßen und erfindungsgemäß bevorzugten Aufhell- oder Färbemittel.

Erfindungsgemäß bevorzugte und bevorzugt verwendete Mittel enthalten, jeweils bezogen auf das Gewicht dieses Mittels, mindestens ein Öl in einer Gesamtmenge von 0,01 - 7 Gew.-%, weiter bevorzugt 0,1 - 6 Gew.-%, besonders bevorzugt 0,5- 5,5 Gew.-%, außerordentlich bevorzugt 1 - 5,2 Gew.-%, wobei diese Mengenangaben die Öle aus der optional enthaltenen, erfindungsgemäß bevorzugten Natriumpolyacrylat-Emulsion einschließen. Dieses zusätzliche Öl kann aus denselben Ölen ausgewählt sein, die auch in den erfindungsgemäß bevorzugten Natriumpolyacrylat-Emulsionen enthalten sein können. Bevorzugt ist das mindestens eine zusätzliche Öl ausgewählt aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, Cs-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen, den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen. Erfindungsgemäß besonders bevorzugte Öle sind ausgewählt aus Paraffinölen, natürlichen Ölen, insbesondere Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannisbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls, weiterhin synthetische Triglyceridöle, insbesondere Capric/ Caprylic Triglycerides, weiterhin die Ester linearer oder verzweigter gesättigter oder ungesättigter Fettalkohole mit 2 - 30 Kohlenstoffatomen mit linearen oder verzweigten gesättigten oder ungesättigten Fettsäuren mit 2 - 30 Kohlenstoff-atomen, die hydroxyliert sein können, insbesondere Isopropylpalmitat und Isopropylmyristat, sowie Mischungen der vorgenannten Öle.

Für die erfindungsgemäße Ausführungsform als Haarfärbemittel ist ein weiteres wesentliches Merkmal der Gehalt an mindestens einem Oxidationsfarbstoffvorprodukt als farbgebender Substanz.

Oxidationsfarbstoffvorprodukte können aufgrund ihres Reaktionsverhaltens in zwei Kategorien eingeteilt werden, so genannte Entwicklerkomponenten, die auch als Oxidationsbasen bezeichnet werden, und Kupplerkomponenten.

Kupplerkomponenten bilden im Rahmen der oxidativen Färbung allein keine signifikante Färbung aus, sondern benötigen stets die Gegenwart von Entwicklerkomponenten. Entwicklerkomponenten können mit sich selbst den eigentlichen Farbstoff ausbilden.

Viele der Entwickler- und Kupplerkomponenten können in freier Form eingesetzt werden. Bei Substanzen mit Aminogruppen kann es aber bevorzugt sein, sie in Salzform, insbesondere in Form der Hydrochloride oder Hydrobromide oder der Sulfate einzusetzen.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe, bestehend aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Besonders bevorzugte Entwicklerkomponenten sind ausgewählt aus p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin und/oder 4,5-Diamino-1-(2-hydroxyethyl)-pyrazol und deren physiologisch verträglichen Salze sowie Mischungen hiervon.

Besonders geeignete Oxidationsfarbstoffvorprodukte vom Kuppler-Typ sind ausgewählt aus der Gruppe, bestehend aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol (2-Amino-4-[(2-hydroxyethyl)amino]-anisol), 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]-ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugte Kupplerkomponenten sind ausgewählt aus 3-Aminophenol, 5-Amino-2-methylphenol, 3-Amino-2-chlor-6-methylphenol, 2-Hydroxy-4-aminophenoxyethanol, 2-(2,4-Diaminophenoxy)ethanol, 1-Methoxy-2-amino-4-(2-hydroxyethylamino)benzol (2-Amino-4-[(2-hydroxyethyl)amino]-anisol), Resorcin, 2-Methylresorcin, 4-Chlorresorcin, 2-Amino-3-hydroxypyridin, und deren physiologisch verträglichen Salzen sowie Mischungen hiervon.

In einer bevorzugten Ausführungsform enthalten die erfindungsgemäßen Färbemittel ein oder mehrere Oxidationsfarbstoffvorprodukte in einer Gesamtmenge von 0,005 bis 12 Gew.-%, bevorzugt von 0,1 bis 7 Gew.-%, weiter bevorzugt von 0,5 bis 5 Gew.-%, weiter bevorzugt von 0,7 bis 2,5 Gew.-% und ganz besonders bevorzugt von 1 bis 1,8 Gew.-%, bezogen auf das Gewicht des erfindungsgemäßen Färbemittels bzw. das Gewicht der erfindungsgemäß verwendeten Zusammensetzung (M1).

In einer bevorzugten Ausführungsform enthalten die erfindungsgemäßen Färbemittel ein oder mehrere Oxidationsfarbstoffvorprodukte, ausgewählt aus mindestens einer Entwicklerkomponente und optional mindestens einer Kupplerkomponente, in einer Gesamtmenge von 0,005 bis 12 Gew.-%, bevorzugt von 0,1 bis 7 Gew.-%, weiter bevorzugt von 0,5 bis 5 Gew.-%, weiter bevorzugt von 0,7 bis 2,5 Gew.-% und ganz besonders bevorzugt von 1 bis 1,8 Gew.-%, bezogen auf das Gewicht des erfindungsgemäßen Färbemittels bzw. das Gewicht der erfindungsgemäß verwendeten Zusammensetzung (M1).

Bezogen auf jedes einzelne Oxidationsfarbstoffvorprodukt, ist dieses bevorzugt in einer Menge von 0,005 - 5 Gew.-%, besonders bevorzugt 0,01 bis 4 Gew.-%, außerordentlich bevorzugt 0,1 - 2 Gew.-%, jeweils bezogen auf das Gewicht des Mittels, enthalten.

In einer weiteren bevorzugten Ausführungsform der Erfindung enthält das erfindungsgemäße Mittel mindestens einen direktziehenden Farbstoff.

In oxidativen Haaraufhellmitteln und Haarfärbemitteln für helle Nuancen dienen direktziehende Farbstoffe häufig zur Nuancierung unerwünschter Rotstiche, die von den Melaninabbauprodukten erzeugt werden können, oder zur Nuancierung bestimmter Blondtöne.

Um eine ausgewogene und subtile Nuancenausbildung zu erhalten, kann es im Rahmen der vorliegenden Erfindung auch vorgesehen sein, dass die kosmetischen Mittel mit OFV zusätzlich mindestens einen direktziehenden Farbstoff enthalten.

Bei direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das kosmetische Mittel mindestens einen direktziehenden Farbstoff in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 2 Gew.-%, jeweils bezogen auf das Gewicht des kosmetischen Mittels bzw. der erfindungsgemäß verwendeten Zusammensetzung (M1).

Überraschend wurde gefunden, dass ein Zusatz an 4-Hydroxyacetophenon das Färbe- bzw. das Aufhellergebnis verbessert. Weitere erfindungsgemäß bevorzugte Haaraufhell- oder Haarfärbemittel sind daher dadurch gekennzeichnet, dass sie 4-Hydroxyacetophenon enthalten. Bevorzugt enthält das kosmetische Mittel 0,001 bis 2 Gew.-%, vorzugsweise von 0,01 bis 1 Gew.-%, bevorzugt von 0,1 bis 0,6 Gew.-%, insbesondere von 0,2 bis 0,4 Gew.-%, 4-Hydroxyacetophenon, jeweils bezogen auf das Gewicht des kosmetischen Mittels bzw. der erfindungsgemäß verwendeten Zusammensetzung (M1).

Ein weiterer Gegenstand der vorliegenden Erfindung ist eine Verpackungseinheit (Kit-of-Parts), die
- getrennt voneinander verpackt - folgendes umfasst:
   a) mindestens einen Container (C1), enthaltend ein Mittel zur oxidativen Haarfärbung, das, jeweils bezogen auf sein Gewicht, Folgendes enthält:
      - 70 - 90 Gew.-%, bevorzugt 73 - 86 Gew.-%, besonders bevorzugt 76 - 84 Gew.-%, Wasser,
      - mindestens ein Alkalisierungsmittel,
      - mindestens ein vernetztes Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, enthalten ist,
      - mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%,
      - mindestens ein anionisches Tensid in einer Gesamtmenge von 1 - 6 Gew.-%, bevorzugt 2 - 5 Gew.-%, und besonders bevorzugt 2,5 - 4,5 Gew.-%,
      - mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%, und
      - Natriumpolyacrylat, bevorzugt mit einer massenmittleren Molmasse M_{w} im Bereich von 1.000.000 bis 20.000.000 Dalton, besonders bevorzugt 6.000.000 bis 15.000.000 Dalton, enthalten ist, bevorzugt in einer Gesamtmenge von 0,1 - 1,5 Gew.-%, besonders bevorzugt 0,5 - 1,3 Gew.-%, außerordentlich bevorzugt 0,8 - 1,1 Gew.-%, jeweils bezogen auf das Gewicht des Mittels, wobei besonders bevorzugt das Natriumpolyacrylat als in einer Wasser-in-ÖI-Emulsion vorgeliert enthalten ist,
      - optional mindestens eine färbende Substanz, ausgewählt aus Oxidationsfarbstoffvorprodukten, direktziehenden Haarfarbstoffen und Mischungen hiervon,
      wobei keine Oxidationsmittel enthalten sind, und
   b) mindestens einen Container (C2), enthaltend eine Oxidationsmittelzubereitung (M2), die 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-% enthält und einen pH-Wert im Bereich von 2,0 bis 6,5, bevorzugt 2,5 - 5,5, besonders bevorzugt 2,8 bis 5,0, aufweist, jeweils gemessen bei 20°C, wobei optional mindestens ein Copolymer, ausgewählt aus vernetzten Acrylsäure/ Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, enthalten sein kann, wobei sich die Gew.-%-Angaben jeweils auf das Gewicht der Oxidationsmittelzubereitung (M2) beziehen.

Ein weiterer Gegenstand der vorliegenden Erfindung ist ein Verfahren zur oxidativen Haarfärbung, das die folgenden Verfahrensschritte umfasst:
i) Bereitstellen eines kosmetischen Mittels (M1) zur oxidativen Haarfärbung keratinischer Fasern, enthaltend
   - 70 - 90 Gew.-%, bevorzugt 73 - 86 Gew.-%, besonders bevorzugt 76 - 84 Gew.-%, Wasser,
   - mindestens ein Alkalisierungsmittel,
   - mindestens ein vernetztes Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, enthalten ist,
   - mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%,
   - mindestens ein anionisches Tensid in einer Gesamtmenge von 1 - 6 Gew.-%, bevorzugt 2 - 5 Gew.-%, und besonders bevorzugt 2,5 - 4,5 Gew.-%,
   - mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%, und
   - Natriumpolyacrylat, bevorzugt mit einer massenmittleren Molmasse M_{w} im Bereich von 1.000.000 bis 20.000.000 Dalton, besonders bevorzugt 6.000.000 bis 15.000.000 Dalton, enthalten ist, bevorzugt in einer Gesamtmenge von 0,1 - 1,5 Gew.-%, besonders bevorzugt 0,5 - 1,3 Gew.-%, außerordentlich bevorzugt 0,8 - 1,1 Gew.-%, jeweils bezogen auf das Gewicht des Mittels, wobei besonders bevorzugt das Natriumpolyacrylat als in einer Wasser-in-ÖI-Emulsion vorgeliert enthalten ist,
   - optional mindestens eine färbende Substanz, ausgewählt aus Oxidationsfarbstoffvorprodukten, direktziehenden Haarfarbstoffen und Mischungen hiervon,
   wobei keine Oxidationsmittel enthalten sind, und
ii) Bereitstellen einer Oxidationsmittelzubereitung (M2), enthaltend 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-% und einen pH-Wert im Bereich von 2,0 bis 6,5, bevorzugt 2,5 - 5,5, besonders bevorzugt 2,8 bis 5,0, aufweisend, jeweils gemessen bei 20°C, wobei optional mindestens ein Copolymer, ausgewählt aus vernetzten Acrylsäure/ Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), enthalten sein kann,
iii) Vermischen des kosmetischen Mittels (M1) mit der Oxidationsmittelzubereitung (M2), bevorzugt in einem Gewichtsverhältnis (M1):(M2) im Bereich von 0,5:1: bis 3:1, bevorzugt 0,8:1 bis 2,5:1 , besonders bevorzugt 1:1 bis 1,5:1, direkt anschließend
iv) Applizieren der in Schritt iii) erhaltenen Mischung auf die Haare und Belassen dieser Mischung für eine Zeit von 1 bis 60 Minuten, bevorzugt von 20 bis 45 Minuten, bei Raumtemperatur und/oder bei 30 - 60°C auf den Haaren,
v) Spülen der Haare mit Wasser und/oder einer reinigenden Zusammensetzung, und
vi) gegebenenfalls Applizieren eines Nachbehandlungsmittels auf die Haare und gegebenenfalls Ausspülen, anschließend Trocknen.

Für oxidative Haaraufhell- oder Haarfärbeverfahren wird üblicherweise unmittelbar vor der Applikation auf das Haar das erfindungsgemäße Mittel (M1), das optional ein oder mehrere Oxidationsfarbstoffvorprodukte und ggf. ein oder mehrere direktziehende Farbstoffe enthält, mit einer wässrigen Oxidationsmittel-haltigen Zusammensetzung (M2) zu dem anwendungsbereiten Färbemittel vermischt und dann auf das Haar appliziert. Meistens sind das erfindungsgemäße Mittel (M1) und die Oxidationsmittel-haltige Zusammensetzung (M2) so aufeinander abgestimmt, dass bei einem Mischungsverhältnis von 1 zu 1, bezogen auf Gewichtsteile, in der fertigen Anwendungsmischung eine Anfangskonzentration an Wasserstoffperoxid von 0,5 - 12 Gew.-%, bevorzugt 0,9 - 7 Gew.-%, besonders bevorzugt 1,5 - 5 Gew.-%, außerordentlich 2,5 - 4 Gew.-% Wasserstoffperoxid (berechnet als 100%iges H₂O₂), jeweils bezogen auf das Gewicht der Anwendungsmischung, vorliegt. Es ist aber genauso gut möglich, das erfindungsgemäße Mittel (M1) und die Oxidationsmittel-haltige Zusammensetzung (M2) so aufeinander abzustimmen, dass sich die im anwendungsbereiten Oxidationsfärbemittel (Anwendungsmischung) notwendigen Konzentrationen, wie vorstehend angegeben, durch andere Mischungsverhältnisse als 1:1 ergeben, beispielsweise durch ein Gewichtsbezogenes Mischungsverhältnis von 5:2 oder 1:2 oder 1:3 oder auch 2:3. Erfindungsgemäß bevorzugte Gewichts-bezogene Mischungsverhältnisse (M1):(M2) liegen im Bereich von 0,5:1: bis 3:1, bevorzugt 0,8:1 bis 2,5:1 , besonders bevorzugt 1:1 bis 1,5:1.

Erfindungsgemäß bevorzugte oxidative Haaraufhell- oder Haarfärbeverfahren sind dadurch gekennzeichnet, dass die fertige Anwendungsmischung aus (M1) und (M2) einen pH-Wert im Bereich von 8,5 - 11,5, bevorzugt 9 - 11, besonders bevorzugt 9,5 - 10,5, aufweist, jeweils gemessen bei 20°C.

Erfindungsgemäß bevorzugte oxidative Haaraufhell- oder Haarfärbe-Kits sind dadurch gekennzeichnet, dass die fertige Anwendungsmischung aus (M1) und (M2) einen pH-Wert im Bereich von 8,5 - 11,5, bevorzugt 9 - 11, besonders bevorzugt 9,5 - 10,5, aufweist, jeweils gemessen bei 20°C.

Der Begriff "Raumtemperatur" bezeichnet erfindungsgemäß die Temperatur in dem Raum, in dem eine Person üblicherweise ein Haaraufhell- oder -färbemittel benutzt, also üblicherweise ein Badezimmer oder ein Friseursalon, in dem eine Temperatur im Bereich von 10 - 29 °C herrscht.

Das Belassen der Haaraufhellenden oder Haarfärbenden Anwendungsmischung in Verfahrensschritt iv) in den erfindungsgemäßen oder erfindungsgemäß bevorzugten Haaraufhell- oder -färbeverfahren kann auch bei mindestens 30 °C, bevorzugt bei 30 - 60°C, besonders bevorzugt bei 32 - 50°C stattfinden, wenn das Haar beispielsweise mit einer Wärmehaube oder mit einem Wärmestrahler erwärmt wird.

Die in erfindungsgemäßen und erfindungsgemäß bevorzugten Färbekits sowie in erfindungsgemäßen und erfindungsgemäß bevorzugten Färbeverfahren eingesetzte Oxidationsmittelzubereitung (M2) enthält, jeweils bezogen auf ihr Gewicht, 40 - 96 Gew.-%, bevorzugt 70 - 93 Gew.-%, besonders bevorzugt 80 - 90 Gew.-%, Wasser.

Die in erfindungsgemäßen und erfindungsgemäß bevorzugten Färbekits sowie in erfindungsgemäßen und erfindungsgemäß bevorzugten Färbeverfahren eingesetzte Oxidationsmittelzubereitung (M2) enthält weiterhin, jeweils bezogen auf ihr Gewicht, 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-% Wasserstoffperoxid.

Zur Stabilisierung des Wasserstoffperoxids weist die Oxidationsmittelzubereitung (M2) einen pH-Wert im Bereich von 2,0 bis 6,5, bevorzugt 2,5 - 5,5, besonders bevorzugt 2,8 bis 5,0, auf, jeweils gemessen bei 20°C.

Die Viskosität erfindungsgemäß bevorzugter Mittel (M1), die im Bereich von 8000 - 40.000 mPas, bevorzugt 10.000 - 35.000 mPas, besonders bevorzugt 12.000 - 30.000 mPas, liegt, jeweils gemessen bei 20°C mit einem Haake VT 550-Viskosimeter, Messsystem SV, liegt, ist für die Handhabung dieses Mittels selbst (Herstellung, Abfüllung, Dosieren zur Herstellung der Mischung mit der Oxidationsmittelzubereitung) hervorragend geeignet. Die Oxidationsmittelzubereitung (M2) weist üblicherweise eine niedrige Viskosität im Bereich von 10 - 6000 mPas, bevorzugt 200 - 5000 mPas, besonders bevorzugt 1000 - 4500 mPas, jeweils gemessen bei 20°C, auf. Für die Applikation auf das Haar allerdings sollte die Anwendungsmischung eine deutlich höhere Viskosität aufweisen, damit sie während der gesamten Einwirkzeit (im Bereich von 5 - 60 Minuten, bevorzugt 30 - 45 Minuten) auf dem Haar verbleibt und nicht heruntertropft. Hierbei unterscheidet man, ob die Anwendungsmischung durch das Schütteln beider Zusammensetzungen (M1) und (M2) in einer Applikationsflasche hergestellt wird, aus der heraus die Anwendungsmischung unverzüglich nach dem Mischen mit Hilfe einer Applikationstülle als Flaschenaufsatz auf das Haar appliziert wird (Flaschenapplikation), oder ob die Anwendungsmischung durch Verrühren beider Zusammensetzungen (M1) und (M2) in einer Schale hergestellt wird, aus der heraus die Anwendungsmischung unverzüglich nach dem Mischen mit einem Pinsel auf das Haar appliziert wird (Pinselapplikation). Die Flaschenapplikation ist insbesondere für Färbemittel geeignet, die im Einzelhandel mit einer Empfehlung zur Anwendung durch den Verbraucher selbst vertrieben werden. Die Pinselapplikation ist insbesondere für Aufhell- und Färbemittel geeignet, die im Friseursalon durch den Friseur hergestellt und auf die Haare des Verbrauchers appliziert werden.

Überraschenderweise wurde gefunden, dass man eine Anwendungsmischung mit einer insbesondere für die Flaschenapplikation geeigneten Viskosität erhält, wenn man das erfindungsgemäße oder erfindungsgemäß bevorzugte Mittel (M1) mit einer Oxidationsmittelzubereitung (M2) vermischt, die mindestens ein Niotensid in einer Gesamtmenge von 0,05 - 2 Gew.-%, bevorzugt 0,3 - 1,5 Gew.-%, weiterhin mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol), 1-Eicosanol (Arachylalkohol) und 1- Docosanol (Behenylalkohol) sowie Mischungen hiervon, in einer Gesamtmenge von 1 - 5 Gew.-%, bevorzugt 1,5 - 4 Gew.-%, und weiterhin mindestens ein Öl in einer Gesamtmenge von 0,2 - 10 Gew.-%, bevorzugt 1 - 5 Gew.-%, besonders bevorzugt 2 - 4 Gew.-%, enthält, wobei sich alle Mengenangaben auf das Gewicht der Oxidationsmittelzubereitung (M2) beziehen.

Besonders bevorzugt ist es, dass diese Oxidationsmittelzubereitung (M2) kein Kationtensid und kein Polymer mit einem Polymerisierungsgrad von mindestens 200 und kein Polymer mit einem Molekulargewicht von 10000 Dalton oder höher enthält.

Die nichtionischen Tenside, die in den erfindungsgemäß verwendeten Oxidationsmittelzubereitungen (M2) eingesetzt werden, sind aus denselben Tensiden ausgewählt, aus denen auch die in den erfindungsgemäßen und erfindungsgemäß verwendeten Mitteln (M1) enthaltenen Niotenside ausgewählt sind.

Im Sinne der vorliegenden Anmeldung werden die vorgenannten linearen, gesättigten 1-Alkanole mit einer Hydroxygruppe, auch in Bezug auf die Oxidationsmittelzubereitungen (M2) nicht zu den Tensiden gezählt.

Das mindestens eine Öl, das in der Oxidationsmittelzubereitung (M2) in einer Gesamtmenge von 0,2 - 10 Gew.-%, bevorzugt 1 - 5 Gew.-%, besonders bevorzugt 2 - 4 Gew.-%, jeweils bezogen auf das Gewicht der Zubereitung (M2), enthalten ist, ist bevorzugt ausgewählt aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen; den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃-₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen. In diesem Zusammenhang erfindungsgemäß besonders bevorzugte Öle sind ausgewählt aus Paraffinölen und den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, sowie Mischungen hiervon; außerordentlich bevorzugt ausgewählt aus Paraffinöl, Isopropylpalmitat und Isopropylmyristat sowie Mischungen hiervon.

Das Vermischen des erfindungsgemäßen oder erfindungsgemäß bevorzugten Mittels mit einer solchen Oxidationsmittelzubereitung (M2) führt zur gewünschten Anwendungsviskosität und damit zu optimalen Anwendungseigenschaften. Die so erzielten Anwendungsmischungen, insbesondere bei Gewichts-bezogenen Mischungsverhältnissen (M1):(M2) im Bereich von 0,5:1: bis 3:1, bevorzugt 0,8:1 bis 2,5:1 , besonders bevorzugt 1:1 bis 1,5:1, weisen bevorzugt eine Viskosität im Bereich von 3.000 - 20.000 mPas, bevorzugt 5.000 - 15.000 mPas, besonders bevorzugt 7.000 - 10.000 mPas, auf, jeweils gemessen bei 20°C (Rotationsviskosimeter Haake VT 550, Messsystem MV II).

Eine weitere erfindungsgemäß bevorzugte Verpackungseinheit (Kit-of-Parts) ist daher dadurch gekennzeichnet, dass die Oxidationsmittelzubereitung (M2) mindestens ein Niotensid in einer Gesamtmenge von 0,05 - 2 Gew.-%, bevorzugt 0,3 - 1,5 Gew.-%, weiterhin mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol), 1-Eicosanol (Arachylalkohol) und 1-Docosanol (Behenylalkohol) sowie Mischungen hiervon, in einer Gesamtmenge von 1 - 5 Gew.-%, bevorzugt 1,5 - 4 Gew.-%, und weiterhin mindestens ein Öl in einer Gesamtmenge von 0,2 - 10 Gew.-%, bevorzugt 1 - 5 Gew.-%, besonders bevorzugt 2 - 4 Gew.-%, enthält, wobei sich alle Mengenangaben auf das Gewicht der Oxidationsmittelzubereitung (M2) beziehen und wobei es besonders bevorzugt ist, dass diese Oxidationsmittelzubereitung (M2) kein Kationtensid und kein Polymer mit einem Polymerisierungsgrad von mindestens 200 und kein Polymer mit einem Molekulargewicht von 10000 Dalton oder höher enthält.

In einer anderen Ausführungsform der Erfindung kann es bevorzugt sein, wenn man das erfindungsgemäße oder erfindungsgemäß bevorzugte Mittel (M1) mit einer Oxidationsmittelzubereitung (M2) vermischt, die mindestens ein Copolymer, ausgewählt aus vernetzten Acrylsäure/ Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), enthält. Das Vermischen des erfindungsgemäßen oder erfindungsgemäß bevorzugten Mittels mit einer solchen Oxidationsmittelzubereitung (M2) führt zur gewünschten Anwendungsviskosität und damit zu optimalen Anwendungseigenschaften. Die so erzielten Anwendungsmischungen, insbesondere bei Gewichts-bezogenen Mischungsverhältnissen (M1):(M2) im Bereich von 0,5:1: bis 3:1, bevorzugt 0,8:1 bis 2,5:1 , besonders bevorzugt 1:1 bis 1,5:1, weisen bevorzugt eine Viskosität im Bereich von 3.000 - 20.000 mPas, bevorzugt 5.000 - 15.000 mPas, besonders bevorzugt 8.000 - 12.000 mPas, auf, jeweils gemessen bei 20°C (Rotationsviskosimeter Haake VT 550, Messsystem MV II).

Eine weitere erfindungsgemäß bevorzugte Verpackungseinheit (Kit-of-Parts) ist daher dadurch gekennzeichnet, dass die Oxidationsmittelzubereitung (M2) mindestens ein Copolymer, ausgewählt aus vernetzten Acrylsäure/ Acrylsäure-C1-C6-Alkylester-Copolymeren und vernetzten Methacrylsäure/ Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und bevorzugt kein Kationtensid enthält.

Ein weiteres erfindungsgemäß bevorzugtes Verfahren zur oxidativen Haaraufhellung oder Haarfärbung ist daher dadurch gekennzeichnet, dass die Oxidationsmittelzubereitung (M2) mindestens ein Copolymer, ausgewählt aus vernetzten Acrylsäure/ Acrylsäure-C1-C6-Alkylester-Copolymeren und vernetzten Methacrylsäure/ Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und bevorzugt kein Kationtensid enthält.

Bevorzugte vernetzte Copolymere dieser Art sind ausgewählt aus - jeweils vernetzten - Methacrylsäure/Methylacrylat-, Methacrylsäure/Ethylacrylat-, Methacrylsäure/Propylacrylat-, Methacrylsäure/ Butylacrylat-, Methacrylsäure/Pentylacrylat-, Methacrylsäure/Hexylacrylat-, Acrylsäure/Methylacrylat-, Acrylsäure/Ethylacrylat-, Acrylsäure/Propylacrylat-, Acrylsäure/Butylacrylat-, Acrylsäure/Pentylacrylat- und Acrylsäure/Hexylacrylat- Copolymeren und Mischungen davon.

Eine weitere erfindungsgemäß bevorzugte Verpackungseinheit (Kit-of-Parts) ist dadurch gekennzeichnet, dass die Oxidationsmittelzubereitung (M2) mindestens ein vernetztes Copolymer, ausgewählt aus - jeweils vernetzten - Methacrylsäure/Methylacrylat-, Methacrylsäure/Ethylacrylat-, Methacrylsäure/ Propylacrylat-, Methacrylsäure/ Butylacrylat-, Methacrylsäure/ Pentylacrylat-, Methacrylsäure/ Hexylacrylat-, Acrylsäure/Methylacrylat-, Acrylsäure/Ethylacrylat-, Acrylsäure/Propylacrylat-, Acrylsäure/ Butylacrylat-, Acrylsäure/Pentylacrylat- und Acrylsäure/Hexylacrylat- Copolymeren und Mischungen davon, in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und kein Kationtensid enthält.

Ein weiteres erfindungsgemäß bevorzugtes Verfahren zur oxidativen Haaraufhellung oder Haarfärbung ist dadurch gekennzeichnet, dass die Oxidationsmittelzubereitung (M2) mindestens ein vernetztes Copolymer, ausgewählt aus - jeweils vernetzten - Methacrylsäure/Methylacrylat-, Methacrylsäure/ Ethylacrylat-, Methacrylsäure/Propylacrylat-, Methacrylsäure/ Butylacrylat-, Methacrylsäure/ Pentylacrylat-, Methacrylsäure/Hexylacrylat-, Acrylsäure/Methylacrylat-, Acrylsäure/Ethylacrylat-, Acrylsäure/Propylacrylat-, Acrylsäure/Butylacrylat-, Acrylsäure/Pentylacrylat- und Acrylsäure/Hexylacrylat-Copolymeren und Mischungen davon, in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und kein Kationtensid enthält.

Die in weiteren erfindungsgemäß bevorzugten Aufhell- oder Färbekits sowie in erfindungsgemäß bevorzugten Aufhell- oder Färbeverfahren eingesetzte Oxidationsmittelzubereitung (M2) enthält mindestens ein Tensid, ausgewählt aus Aniontensiden und Niotensiden sowie Mischungen hiervon, in einer Gesmtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol), 1-Eicosanol (Arachylalkohol) und 1-Docosanol sowie Mischungen hiervon, in einer Gesamtmenge von 1 - 5 Gew.-%, bevorzugt 1,5 - 4 Gew.-%, enthält, wobei sich alle Mengenangaben auf das Gewicht der Oxidationsmittelzubereitung (M2) beziehen.

Die anionischen Tenside und die nichtionischen Tenside, die in den erfindungsgemäß verwendeten Oxidationsmittelzubereitungen (M2) eingesetzt werden, sind aus denselben Tensiden ausgewählt, aus denen auch die in den erfindungsgemäßen und erfindungsgemäß verwendeten Mitteln (M1) enthaltenen Anion- und Niotenside ausgewählt sind.

Im Sinne der vorliegenden Anmeldung werden die vorgenannten linearen, gesättigten 1-Alkanole mit einer Hydroxygruppe, auch in Bezug auf die Oxidationsmittelzubereitungen (M2) nicht zu den Tensiden gezählt.

In einer weiteren bevorzugten Ausführungsform der Erfindung enthält die erfindungsgemäß eingesetzte Oxidationsmittelzubereitung (M2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2). Das mindestens eine Öl, das in der Oxidationsmittelzubereitung (M2) in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Zubereitung (M2), enthalten ist, ist bevorzugt ausgewählt aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen; den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen. In diesem Zusammenhang erfindungsgemäß besonders bevorzugte Öle sind ausgewählt aus Paraffinölen und den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, sowie Mischungen hiervon; außerordentlich bevorzugt ausgewählt aus Paraffinöl, Isopropylpalmitat und Isopropylmyristat sowie Mischungen hiervon.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), aber kein Kationtensid enthält.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), aber kein Polymer mit einem Polymerisierungsgrad von mindestens 200 und kein Polymer mit einem Molekulargewicht von 10000 Dalton oder höher enthält.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), aber kein Kationtensid, kein Polymer mit einem Polymerisierungsgrad von mindestens 200 und kein Polymer mit einem Molekulargewicht von 10000 Dalton oder höher enthält.

In einer weiteren bevorzugten Ausführungsform der Erfindung wurde gefunden, dass man eine Anwendungsmischung mit einer insbesondere für die Pinselapplikation geeigneten Viskosität erhält, wenn man das erfindungsgemäße oder erfindungsgemäß bevorzugte Mittel (M1) mit einer Oxidationsmittelzubereitung (M2) vermischt, die mindestens ein Kationtensid enthält. Beim Vermischen führt die Wechselwirkung zwischen dem mindestens einen vernetzten Copolymer aus Acrylsäure und nicht-ethoxylierten Estern der Acrylsäure mit linearen C10-C30-Monoalkoholen und dem mindestens einen Kationtensid zum gewünschten Anstieg der Viskosität. Die damit erzielte Konsistenz der Anwendungsmischung führt zu optimalen Anwendungseigenschaften.

In einer weiteren bevorzugten Ausführungsform der Erfindung enthält die erfindungsgemäß eingesetzte Oxidationsmittelzubereitung (M2) mindestens ein Kationtensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (M2). Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Mizellbildungskonzentration zu positiv geladenen Mizellen.

Erfindungsgemäß bevorzugt sind kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride, Trialkylmethylammoniumchloride, sowie die unter den INCI-Bezeichnungen Quaternium-27 und Quaternium-83 bekannten Imidazolium-Verbindungen. Weitere bevorzugte quaternäre Ammoniumverbindungen sind Tetraalkylammoniumsalze, wie insbesondere das unter der INCI-Bezeichnung bekannte Quaternium-52, ein Poly(Oxy-1,2-Ethanediyl), ((Octadecylnitrilio)tri-2,1-Ethanediyl)tris(Hydroxy)-Phosphat (1:1)-Salz, das die allgemeine Strukturformel (III) aufweist, worin x + y + z = 10 sind:

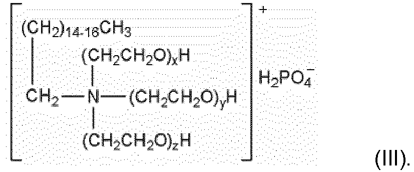

Die langen Alkylketten der oben genannten Tenside weisen bevorzugt 10 bis 22, besonders bevorzugt 12 bis 18 Kohlenstoffatome auf. Besonders bevorzugt sind Behenyltrimethylammoniumchlorid, Stearyltrimethylammoniumchlorid und Cetyltrimethylammoniumchlorid, wobei Stearyltrimethylammoniumchlorid außerordentlich bevorzugt ist. Weitere erfindungsgemäß geeignete kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tego-amid^{®} S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben.

In Bezug auf optimale Anwendungseigenschaften und optimale Aufhell- oder Färbeergebnisse haben sich C10-C22-Alkyltrimethylammoniumchloride als besonders gut geeignet herausgestellt. Besonders bevorzugte erfindungsgemäß verwendete Oxidationsmittelzubereitungen (M2) sind daher dadurch gekennzeichnet, dass sie mindestens ein kationisches Tensid in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (M2), enthalten, wobei bevorzugt mindestens ein Tensid, ausgewählt aus C10-C22-Alkyltrimethylammoniumchloriden, insbesondere ausgewählt aus Behenyltrimethylammoniumchlorid, Stearyltrimethylammoniumchlorid und Cetyltrimethylammoniumchlorid sowie Mischungen dieser Tenside, enthalten sind. Außerordentlich bevorzugte erfindungsgemäß verwendete Oxidationsmittelzubereitungen (M2) enthalten Stearyltrimethylammoniumchlorid in einer Gesamtmenge von 0,05 - 3 Gew.-%, besonders bevorzugt von 0,1 - 1,5 Gew.-%, außerordentlich bevorzugt 0,3 - 0,9 Gew.-%, jeweils bezogen auf das Gewicht der Oxidationsmittelzubereitung (M2).

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein kationisches Tensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (M2), enthält.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein kationisches Tensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (M2), aber kein Polymer mit einem Polymerisierungsgrad von mindestens 200 und kein Polymer mit einem Molekulargewicht von 10000 Dalton oder höher enthält.

Es wurde gefunden, dass die Verdickung mit Hilfe der Wechselwirkung zwischen dem Copolymer in dem erfindungsgemäßen Mittel und dem Kationtensid in der Oxidationsmittelzubereitung (M2) ausreichend ist und durch die Gegenwart eines Polymers mit einem Polymerisierungsgrad von mindestens 200 oder eines Polymers mit einem Molekulargewicht von 10000 Dalton oder höher nicht weiter gesteigert bzw. sogar in ihren Anwendungseigenschaften beeinträchtigt sein kann.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, jeweils bezogen auf das Gewicht der Oxidationsmittelzubereitung (M2), enthält.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol), 1-Eicosanol (Arachylalkohol) und 1-Docosanol sowie Mischungen hiervon, in einer Gesamtmenge von 1 - 5 Gew.-%, bevorzugt 1,5 - 4 Gew.-%, jeweils bezogen auf das Gewicht der Oxidationsmittelzubereitung (M2), aber kein Polymer mit einem Polymerisierungsgrad von mindestens 200 und kein Polymer mit einem Molekulargewicht von 10000 Dalton oder höher enthält.

Es wurde gefunden, dass die Verdickung mit Hilfe der Wechselwirkung zwischen dem Copolymer in dem erfindungsgemäßen Mittel und der vorgenannten Tensid/1-Alkanol-Mischung in der Oxidationsmittelzubereitung (M2) ausreichend ist und durch die Gegenwart eines Polymers mit einem Polymerisierungsgrad von mindestens 200 oder eines Polymers mit einem Molekulargewicht von 10000 Dalton oder höher nicht weiter gesteigert bzw. sogar in ihren Anwendungseigenschaften beeinträchtigt sein kann.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, und mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), enthält.

Eine weitere erfindungsgemäß bevorzugte Verpackungseinheit (Kit-of-Parts) und ein weiteres erfindungsgemäß bevorzugtes Haaraufhell- oder Haarfärbeverfahren sind jeweils dadurch gekennzeichnet, dass die Oxidationsmittelzubereitung (M2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, und mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), aber kein Polymer mit einem Polymerisierungsgrad von mindestens 200 und kein Polymer mit einem Molekulargewicht von 10000 Dalton oder höher enthält.

Die erfindungsgemäß verwendeten und erfindungsgemäß bevorzugt verwendeten Oxidationsmittelzubereitungen (M2) können darüber hinaus Stabilisatoren, insbesondere Komplexbildner, und pH-Puffersubstanzen, enthalten.

Bezüglich des kosmetischen Mittels (M1) in dem Container C1 und der Oxidationsmittelzubereitung (M2) in dem Container C2 der erfindungsgemäßen und erfindungsgemäß bevorzugten Kits gilt mutatis mutandis das zu den erfindungsgemäßen und erfindungsgemäß bevorzugten kosmetischen Mitteln Gesagte.

Bezüglich des kosmetischen Mittels (M1) in dem Container C1 der erfindungsgemäßen und erfindungsgemäß bevorzugten Verfahren zur oxidativen Haaraufhellung oder Haarfärbung gilt mutatis mutandis das zu den erfindungsgemäßen und erfindungsgemäß bevorzugten kosmetischen Mitteln Gesagte.

Bezüglich der Oxidationsmittelzubereitung (M2) in dem Container C2 der erfindungsgemäßen und erfindungsgemäß bevorzugten Verfahren zur oxidativen Haaraufhellung oder Haarfärbung gilt mutatis mutandis das zu den Oxidationsmittelzubereitungen (M2) der erfindungsgemäßen und erfindungsgemäß bevorzugten Kits zur oxidativen Haaraufhellung oder Haarfärbung Gesagte.

Die Behälterwandung der Container C1 und C2 ist bevorzugt aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polyethylen, insbesondere high density Polyethylen (HDPE), bevorzugt geeignet.

Zur verbesserten Durchmischung von (M1) und (M2) ist es bevorzugt, dass der Container (C2), der die Oxidationsmittelzubereitung (M2) enthält, als Flasche ausgebildet ist und eine wiederverschließbare Öffnung, wie beispielsweise einen Schnapp- oder einen Schraubverschluss, besitzt. Dies ermöglicht die erleichterte Zugabe des farbverändernden Mittels aus Container (C1), der seinerseits bevorzugt in Form einer Flasche aus einem Polyolefin, ausgeführt ist.

Zusammenfassend wird der Gegenstand der vorliegenden Erfindung in folgenden Punkten zusammengefasst:
1. Mittel zur oxidativen Haaraufhellung oder Haarfärbung, enthaltend, jeweils bezogen auf das Gewicht des Mittels,
   - 70 - 90 Gew.-%, bevorzugt 73 - 86 Gew.-%, besonders bevorzugt 76 - 84 Gew.-%, Wasser,
   - mindestens ein Alkalisierungsmittel,
   - mindestens ein vernetztes Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, jeweils bezogen auf das Gewicht des Mittels, enthalten ist,
   - mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%,
   - mindestens ein anionisches Tensid in einer Gesamtmenge von 1 - 6 Gew.-%, bevorzugt 2 - 5 Gew.-%, und besonders bevorzugt 2,5 - 4,5 Gew.-%, und
   - mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%,
   - optional mindestens eine färbende Substanz, ausgewählt aus Oxidationsfarbstoffvorprodukten, direktziehenden Haarfarbstoffen und Mischungen hiervon,
   wobei keine Oxidationsmittel enthalten sind,
   dadurch gekennzeichnet, dass
   - Natriumpolyacrylat, bevorzugt mit einer massenmittleren Molmasse M_{w} im Bereich von 1.000.000 bis 20.000.000 Dalton, besonders bevorzugt 6.000.000 bis 15.000.000 Dalton, enthalten ist, bevorzugt in einer Gesamtmenge von 0,1 - 1,5 Gew.-%, besonders bevorzugt 0,5 - 1,3 Gew.-%, außerordentlich bevorzugt 0,8 - 1,1 Gew.-%, jeweils bezogen auf das Gewicht des Mittels, wobei besonders bevorzugt das Natriumpolyacrylat als in einer Wasser-in-ÖI-Emulsion vorgeliert enthalten ist.
2. Mittel nach Punkt 1, dadurch gekennzeichnet, dass das Alkalisierungsmittel ausgewählt ist aus der Gruppe, umfassend Ammoniumhydroxid, basische Aminosäuren, Alkalihydroxide, Alkanolamine, Alkalimetallmetasilikate, Alkaliphosphate und Alkalihydrogenphosphate sowie Mischungen hiervon, besonders bevorzugt ausgewählt ist aus der Gruppe, umfassend Alkanolamine, Kaliumhydroxid, Natriumhydroxid sowie Mischungen hiervon.
3. Mittel nach Punkt 1 oder 2, dadurch gekennzeichnet, dass das mindestens eine anionische Tensid ausgewählt ist aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei bevorzugt Natriumlaurethsulfat mit 1 bis 3, besonders bevorzugt 2, Ethylenoxidgruppen im Molekül, enthalten ist.
4. Mittel nach einem der Punkte 1 - 3, dadurch gekennzeichnet, dass das mindestens eine vernetzte Copolymer aus Acrylsäure und nicht-ethoxylierten Estern der Acrylsäure mit linearen C10-C30-Monoalkoholen ausgewählt ist aus Copolymeren mit der INCI-Bezeichnung Acrylates/C10-30 Alkyl Acrylate Crosspolymer.
5. Mittel nach einem der Punkte 1 - 4, gekennzeichnet durch einen pH-Wert im Bereich von 7,5 - 12, bevorzugt 9 - 11, besonders bevorzugt 9,5 - 10,5, jeweils gemessen bei 20°C.
6. Mittel nach einem der Punkte 1 - 5, gekennzeichnet durch eine Viskosität im Bereich von 8000 - 40.000 mPas, bevorzugt 10.000 - 35.000 mPas, besonders bevorzugt 12.000 - 30.000 mPas, liegt, jeweils gemessen bei 20°C mit einem Rotationsviskosimeter Haake VT 550, Messsystem SV.
7. Mittel nach einem der Punkte 1 - 6, dadurch gekennzeichnet, dass das mindestens eine nichtionische Tensid ausgewählt ist aus mit 7 - 80 Mol Ethylenoxid pro Mol ethoxyliertem Rizinusöl, ethoxylierten C₈-C₃₀-Alkanolen mit 6 - 30, bevorzugt 12 bis 20 Mol Ethylenoxid pro Mol, ethoxylierten C₈-C₃₀-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂-C₃₀-Carbonsäuren, die hydroxyliert sein können, Alkylmono- und -oligoglycosiden mit 8 bis 22 Kohlenstoffatomen im Alkylrest, sowie Mischungen der vorgenannten Substanzen.
8. Mittel nach einem der Punkte 1 - 7, dadurch gekennzeichnet, dass das mindestens eine kationische oder zwitterionische Polymer ausgewählt ist aus Polyquaternium-22, Polyquaternium-39, Acrylamidopropyltrimonium chloride/Acrylate Copolymer und Polyquaternium-6 sowie Mischungen hiervon, wobei Polyquaternium-22 besonders bevorzugt ist.
9. Mittel nach einem der Punkte 1 - 8, dadurch gekennzeichnet, dass mindestens ein Öl, bevorzugt in einer Gesamtmenge von 0,01 - 7 Gew.-%, weiter bevorzugt 0,1 - 6 Gew.-%, besonders bevorzugt 0,5- 5,5 Gew.-%, außerordentlich bevorzugt 1 - 5,2 Gew.-%, jeweils bezogen auf das Gewicht des Mittels, enthalten ist.
10. Mittel nach einem der Punkte 1 - 9, dadurch gekennzeichnet, dass das mindestens eine lineare, gesättigte 1-Alkanol mit einer Hydroxygruppe und 8 bis 22 Kohlenstoffatomen ausgewählt ist aus Laurylalkohol, Kokosalkohol,1-Tetradecanol (Myristylalkohol), Cetylalkohol und Stearylalkohol sowie Mischungen hiervon, insbesondere aus Kokosalkohol und Laurylalkohol.
11. Mittel nach einem der Punkte 1 - 10, dadurch gekennzeichnet, dass das mindestens eine nichtionische Tensid in einer Gesamtmenge von 0,01 - 1 Gew.-%, bevorzugt 0,05 - 0,5 Gew.-%, und besonders bevorzugt 0,1 - 0,3 Gew.-%, jeweils bezogen auf das Gewicht des Mittels, enthalten ist.
12. Mittel nach einem der Punkte 1 - 11, dadurch gekennzeichnet, dass das mindestens eine Öl ausgewählt ist aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen, den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.
13. Mittel nach einem der Punkte 1 - 12, dadurch gekennzeichnet, dass mindestens eine farbgebende Substanz, ausgewählt aus Oxidationsfarbstoffvorprodukten, bevorzugt in einer Menge von 0,005 - 5 Gew.-%, bezogen auf das Gewicht des Mittels, enthalten ist, wobei besonders bevorzugt mindestens eine Entwicklerkomponente und optional mindestens eine Kupplerkomponente enthalten ist, wobei außerordentlich bevorzugt die Gesamtmenge an Oxidationsfarbstoffvorprodukten 0,005 - 12 Gew.-%, bevorzugt 0,1 - 7 Gew.-%, besonders bevorzugt 0,5 - 5 Gew.-%, jeweils bezogen auf das Gewicht des Mittels, beträgt.
14. Mittel nach einem der Punkte 1 - 13, dadurch gekennzeichnet, dass mindestens eine farbgebende Substanz, ausgewählt aus direktziehenden Haarfarbstoffen, bevorzugt in einer Gesamtmenge von 0,001 bis 10 Gew.-%, vorzugsweise von 0,01 bis 8 Gew.-%, weiter bevorzugt von 0,1 bis 5 Gew.-%, insbesondere von 0,5 bis 2 Gew.-%, jeweils bezogen auf das Gewicht des Mittels, enthalten ist.
15. Mittel nach einem der Punkte 1 - 14, dadurch gekennzeichnet, dass Polyquaternium-22, bevorzugt in einer Menge von 0,1 - 2 Gew.-%, besonders bevorzugt 0,2 - 1,5 Gew.-%, außerordentlich bevorzugt 0,35 - 0,5 Gew.-%, jeweils bezogen auf das Gewicht des Mittels, enthalten ist.
16. Mittel nach einem der Punkte 1 - 15, dadurch gekennzeichnet, dass 4-Hydroxyacetophenon, bevorzugt in einer Menge von 0,001 bis 2 Gew.-%, vorzugsweise von 0,01 bis 1 Gew.-%, bevorzugt von 0,1 bis 0,6 Gew.-%, insbesondere von 0,2 bis 0,4 Gew.-%, jeweils bezogen auf das Gewicht des Mittels, enthalten ist.
17. Verpackungseinheit (Kit-of-Parts), umfassend - getrennt voneinander verpackt -
   a) mindestens einen Container (C1), enthaltend ein Mittel zur oxidativen Haarfärbung nach einem der Punkte 1 bis 16, und
   b) mindestens einen Container (C2), enthaltend eine Oxidationsmittelzubereitung (M2), die 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-% enthält und einen pH-Wert im Bereich von 2,0 bis 6,5, bevorzugt 2,5 - 5,5, besonders bevorzugt 2,8 bis 5,0, aufweist, jeweils gemessen bei 20°C, wobei sich die Gew.-%-Angaben jeweils auf das Gewicht der Oxidationsmittelzubereitung (M2) beziehen.
18. Verpackungseinheit (Kit-of-Parts) nach Punkt 17, dadurch gekennzeichnet, dass die Oxidationsmittelzubereitung (M2) mindestens ein Copolymer, ausgewählt aus vernetzten Acrylsäure/Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und bevorzugt kein Kationtensid enthält.
19. Verpackungseinheit (Kit-of-Parts) nach Punkt 18, dadurch gekennzeichnet, dass die Oxidationsmittelzubereitung (M2) mindestens ein vernetztes Copolymer, ausgewählt aus - jeweils vernetzten - Methacrylsäure/Methylacrylat-, Methacrylsäure/Ethylacrylat-, Methacrylsäure/Propylacrylat-, Methacrylsäure/Butylacrylat-, Methacrylsäure/Pentylacrylat-, Methacrylsäure/Hexylacrylat-, Acrylsäure/Methylacrylat-, Acrylsäure/Ethylacrylat-, Acrylsäure/Propylacrylat-, Acrylsäure/ Butylacrylat-, Acrylsäure/Pentylacrylat- und Acrylsäure/Hexylacrylat- Copolymeren und Mischungen davon, in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), und kein Kationtensid enthält.
20. Verpackungseinheit (Kit-of-Parts) nach einem der Punkte 17 - 19, dadurch gekennzeichnet, dass die Oxidationsmittelzubereitung (M2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol), 1-Eicosanol (Arachylalkohol) und 1-Docosanol sowie Mischungen hiervon, in einer Gesamtmenge von 1 - 5 Gew.-%, bevorzugt 1,5 - 4 Gew.-%, enthält, wobei sich alle Mengenangaben auf das Gewicht der Oxidationsmittelzubereitung (M2) beziehen.
21. Verpackungseinheit (Kit-of-Parts) nach einem der Punkte 17 - 20, dadurch gekennzeichnet, dass die Oxidationsmittelzubereitung (M2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (M2), enthält.
22. Verpackungseinheit (Kit-of-Parts) nach einem der Punkte 17, 20 oder 21, dadurch gekennzeichnet, dass die Oxidationsmittelzubereitung (M2) mindestens ein Kationtensid enthält, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (M2).
23. Verpackungseinheit (Kit-of-Parts) nach einem der Punkte 17 oder 21 - 22, dadurch gekennzeichnet, dass die Oxidationsmittelzubereitung (M2) kein Polymer mit einem Polymerisierungsgrad von mindestens 200 und kein Polymer mit einem Molekulargewicht von 10000 Dalton oder höher enthält.
24. Verpackungseinheit (Kit-of-Parts) nach einem der Punkte 17 bis 21 oder 23, dadurch gekennzeichnet, dass die Oxidationsmittelzubereitung (M2) keine Kationtenside enthält.
25. Verpackungseinheit (Kit-of-Parts) nach einem der Punkte 17 bis 24, dadurch gekennzeichnet, dass die fertige Anwendungsmischung aus (M1) und (M2) einen pH-Wert im Bereich von 8,5 - 11,5, bevorzugt 9 - 11, besonders bevorzugt 9,5 - 10,5, aufweist, jeweils gemessen bei 20°C.
26. Verfahren zur oxidativen Haaraufhellung oder Haarfärbung, das die folgenden Verfahrensschritte umfasst:
   i) Bereitstellen eines kosmetischen Mittels zur oxidativen Haarfärbung (M1) nach einem der Punkte 1 bis 16,
   ii) Bereitstellen einer Oxidationsmittelzubereitung (M2), enthaltend 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 12 Gew.-% und außerordentlich bevorzugt 6 bis 9 Gew.-%, und einen pH-Wert im Bereich von 2,0 bis 6,5, bevorzugt 2,5 - 5,5, besonders bevorzugt 2,8 bis 5,0, aufweisend, jeweils gemessen bei 20°C, wobei optional mindestens ein Copolymer, ausgewählt aus vernetzten Acrylsäure/Acrylsäure-C1-C6-Alkylester-Copolymeren und vernetzten Methacrylsäure/ Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, und bevorzugt kein Kationtensid enthalten sein kann, wobei sich alle Mengenangaben auf das Gewicht der Oxidationsmittelzubereitung (M2) beziehen,
   iii) Vermischen des kosmetischen Mittels (M1) mit der Oxidationsmittelzubereitung (M2), bevorzugt in einem Gewichtsverhältnis (M1):(M2) im Bereich von 0,5:1: bis 3:1, bevorzugt 0,8:1 bis 2,5:1 , besonders bevorzugt 1:1 bis 1,5:1, direkt anschließend
   iv) Applizieren der in Schritt iii) erhaltenen Mischung auf die Haare und Belassen dieser Mischung für eine Zeit von 1 bis 60 Minuten, bevorzugt von 20 bis 45 Minuten, bei Raumtemperatur und/oder bei 30 - 60°C auf den Haaren,
   v) Spülen der Haare mit Wasser und/oder einer reinigenden Zusammensetzung, und
   vi) gegebenenfalls Applizieren eines Nachbehandlungsmittels auf die Haare und gegebenenfalls Ausspülen, anschließend Trocknen.
27. Verfahren zur oxidativen Haaraufhellung oder Haarfärbung gemäß Punkt 26, dadurch gekennzeichnet, dass die Oxidationsmittelzubereitung (M2) wie in einem der Punkte 18 - 25 ausgebildet ist.
28. Verfahren zur oxidativen Haaraufhellung oder Haarfärbung gemäß Punkt 26 oder 27, dadurch gekennzeichnet, dass die anwendungsbereite Mischung aus (M1) und (M2) eine Viskosität im Bereich von 3.000 - 20.000 mPas, bevorzugt 5.000 - 15.000 mPas, besonders bevorzugt 8.000 - 12.000 mPas, aufweist, jeweils gemessen bei 20°C.

Die nachfolgenden Beispiele sollen den Gegenstand der vorliegenden Erfindung verdeutlichen, ohne ihn hierauf zu beschränken.

**Tabelle 1: Färbecremes für die oxidative Haarfärbung (alle Mengenangaben in Gew.-%)**

| **Inhaltsstoff** | **E1** |
|---|---|
| Octyldodecanol | 4,00 |
| Natriumlaureth(2)-sulfat | 2,70 |
| Monoethanolamin | 2,61 |
| 1-Hydroxyethyl-4,5-diaminopyrazolsulfat | 1,15 |
| Natriumpolyacrylat** | 1,00 |
| Kokosalkohol | 0,75 |
| Paraffinum Liquidum (Mineralöl) | 0,74 |
| m-Aminophenol | 0,45 |
| Polyquaternium-22 | 0,41 |
| Acrylates/C10-30 Alkyl Acrylate Crosspolymer | 0,40 |
| Kaliumhydroxid | 0,25 |
| 4-Hydroxypropylamino-3-nitrophenol | 0,24 |
| 4-Amino-3-nitrophenol | 0,24 |
| Natriumsulfit | 0,20 |
| Traubenkernöl | 0,20 |
| Hydroxyethyl-2-nitro-p-toluidin | 0,18 |
| Etidronsäure | 0,12 |
| Trideceth-6 | 0,10 |
| 4-Am ino-2-hyd roxytoluol | 0,10 |
| Parfüm | 0,12 |
| C11-13 Isoparaffin | 0,06 |
| Wasser | 83,98 |
| | |
| Viskosität [mPas]*** | 12.000 mPas |

| | |
|---|---|
| * Rohstoff "Synative AL T" von BASF; INCI: Coconut alcohol; C10 und kürzer: max. 3 Gew.-%, C12: 48 - 58 Gew.-%, C14: 18-24 Gew.-%, C16: 8-12 Gew.-%, C18: 11-15 Gew.-%, C20: max. 1 Gew.-% ** Natriumpolyacrylat mit Molmasse M_{w} im Bereich von 1.000.000 bis 20.000.000 Dalton, vorgeliert in einer Wasser-in-Mineralöl-Emulsion mit Trideceth-6 als Emulgator *** Viskosität: jeweils gemessen bei 20°C mit einem Rotationsviskosimeter Haake VT 550, Messsystem SV | |

**Tabelle 2: Oxidationsmittel-haltiger Entwickler für die Färbecreme aus Tabelle 1**

| **Inhaltsstoff** | **Einwaage (Gew.-%)** |
|---|---|
| Natriumbenzoat | 0,04 |
| Dinatriumpyrophosphat | 0,30 |
| Na₂-EDTA | 0,15 |
| Phosphorsäure (85 Gew.-%) | 0,04 |
| Natriumcetearylsulfat | 0,20 |
| Cetearylalkohol | 1,70 |
| PEG-40 Castor Oil | 0,40 |
| Wasserstoffperoxid | 9,00 |
| Wasser | ad 100,00 |

| | |
|---|---|
| Viskosität: 2500 mPas, gemessen bei 20°C mit einem Rotationsviskosimeter (Haake VT 550) mit Messsystem MV II | |

**Tabelle 3: Oxidationsmittel-haltiger Entwickler für die Färbecremes aus Tabelle 1**

| **Inhaltsstoff** | **Einwaage (Gew.-%)** |
|---|---|
| Natriumhydroxid | 0,40 |
| Dipicolinsäure (2,6-Dicarboxypyridin) | 0,10 |
| Dinatriumpyrophosphat | 0,03 |
| Etidronsäure | 0,15 |
| Mischung aus vernetzten (Meth)Acrylsäure/ Acrylsäure-C1-C6-Alkylester- | 4,20 (Aktiv) |
| Copolymeren (ex Aculyn 33A) | |
| Natriumlaureth(2)sulfat | 0,50 |
| Wasserstoffperoxid | 6,00 |
| Wasser | ad 100,00 |

| | |
|---|---|
| * Aculyn 33A: wässrige Dispersion von Acrylates Copolymer (Mischung aus vernetzten (Meth)Acrylsäure/ Acrylsäure-C1-C6-Alkylester-Copolymeren); 28 Gew.-% Polymergehalt (Aktivsubstanz) Viskosität: 200 mPas, gemessen bei 20°C mit einem Brookfield-Rotationsviskosimeter bei einer Rotationsfrequenz von 20 min⁻¹ mit Spindel 2 | |

**Tabelle 4: Oxidationsmittel-haltiger Entwickler für die Färbecremes aus Tabelle 1**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,10 |
| 1,2-Propandiol | 0,50 |
| Etidronsäure | 0,15 |
| Paraffinöl | 2,00 |
| Cetearylalkohol | 3,40 |
| Ceteareth-20 | 1,00 |
| Wasserstoffperoxid | 6,00 |
| Wasser | ad 100,00 |

| | |
|---|---|
| Viskosität: 3.500 mPas, gemessen bei 20°C mit einem Rotationsviskosimeter (Haake VT 550) bei einer Rotationsfrequenz von 4 min⁻¹ mit Messgeometrie MV II | |

**Tabelle 5: Oxidationsmittel-haltiger Entwickler für die Färbecremes aus Tabelle 1**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,10 |
| 1,2-Propandiol | 1,00 |
| Etidronsäure | 0,15 |
| Paraffinöl | 0,30 |
| Stearyltrimethylammoniumchlorid | 0,30 |
| Cetearylalkohol | 3,40 |
| Ceteareth-20 | 1,00 |
| Wasserstoffperoxid | 6,00 |
| Wasser | ad 100,00 |

| | |
|---|---|
| Viskosität: 4500 mPas, gemessen bei 20°C mit einem Rotationsviskosimeter (Haake VT 550) bei einer Rotationsfrequenz von 4 min⁻¹ mit Messgeometrie MV II | |

**Tabelle 6: Oxidationsmittel-haltiger Entwickler für die Färbecreme aus Tabelle 1**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,12 |
| Etidronsäure | 0,15 |
| Paraffinöl | 20,00 |
| Natriumcetearylsulfat | 0,36 |
| Cetearylalkohol | 3,50 |
| PEG-40 Castor Oil | 0,70 |
| Wasserstoffperoxid | 6,00 |
| Wasser | ad 100,00 |

| | |
|---|---|
| Viskosität: 7.500 mPas, gemessen bei 20°C mit einem Rotationsviskosimeter (Haake VT 550) bei einer Rotationsfrequenz von 4 min⁻¹ mit Messgeometrie MV II | |

**Tabelle 7: Oxidationsmittel-haltiger Entwickler für die Färbecreme aus Tabelle 1**

| **Inhaltsstoff** | **Einwaage (Gew.-%)** |
|---|---|
| Natriumbenzoat | 0,04 |
| Dinatriumpyrophosphat | 0,10 |
| Kaliumhydroxid | 0,12 |
| Etidronsäure | 0,15 |
| Natriumcetearylsulfat | 0,20 |
| Cetearylalkohol | 1,70 |
| PEG-40 Castor Oil | 0,40 |
| Wasserstoffperoxid | 6,00 |
| Wasser | ad 100,00 |

| | |
|---|---|
| Viskosität: 2500 mPas, gemessen bei 20°C mit einem Rotationsviskosimeter (Haake VT 550) mit Messsystem MV II | |

### Herstellung der Anwendungsmischungen und Ausfärbung auf Haaren

Färbegel und Entwickler gemäß Tabelle 2 wurden im Gewichtsverhältnis 5:2 (5 Gewichtsteile Färbegel auf 2 Gewichtsteile Entwickler) homogen miteinander vermischt. Die so erhaltene Anwendungsmischung wurde unmittelbar nach der Herstellung auf Strähnen menschlichen Haares (Naturweißes Haar, Firma Kerling) aufgetragen (Flottenverhältnis 4 Gramm Anwendungsmischung pro Gramm Haar) und für eine Einwirkzeit von 30 Minuten bei Raumtemperatur (22°C) auf den Haaren belassen. Anschließend wurden die Strähnen ausgespült und mit einem Handtuch getrocknet.

**Tabelle 8: Herstellung der Anwendungsmischungen für die Ausfärbung auf Haaren**

| **alkalische Färbecreme (M1)** | **Entwickler (M2)** | **Gewichtsverhältnis (M1):(M2)** | **Viskosität der Anwendungsmischungen [mPas] **** |
|---|---|---|---|
| gemäß Tabelle 1 | gemäß Tabelle 2 | 5:2 | 12.000 |

| | | | |
|---|---|---|---|
| ** Viskosität: gemessen bei 20°C (Haake-Rotationsviskosimeter VT 550, Messsystem MV II). | | | |

Untersuchung des Einflusses von Polyquaternium-22 auf die Viskosität

**Tabelle 9: Färbecremes für die oxidative Haarfärbung (alle Mengenangaben in Gew.-%)**

| **Inhaltsstoff** | **V1** | **E2** | **E3** | **V2** |
|---|---|---|---|---|
| Octyldodecanol | 4,00 | 4,00 | 4,00 | 4,00 |
| Natriumlaureth(2)-sulfat | 2,70 | 2,70 | 2,70 | 2,70 |
| Monoethanolamin | 2,61 | 2,61 | 2,61 | 2,61 |
| Natriumpolyacrylat** | 1,00 | 1,00 | 1,00 | 1,00 |
| Kokosalkohol | 0,75 | 0,75 | 0,74 | 2,00 |
| Paraffinum Liquidum (Mineralöl) | 0,74 | 0,74 | 0,74 | 0,74 |
| Polyquaternium-22 | - | 0,41 | 0,82 | - |
| Acrylates/C10-30 Alkyl Acrylate Crosspolymer | 0,40 | 0,40 | 0,40 | 0,40 |
| Kaliumhydroxid | 0,25 | 0,25 | 0,25 | 0,25 |
| Natriumsulfit | 0,20 | 0,20 | 0,20 | 0,20 |
| Traubenkernöl | 0,20 | 0,20 | 0,20 | 0,20 |
| Etidronsäure | 0,12 | 0,12 | 0,12 | 0,12 |
| Trideceth-6 | 0,10 | 0,10 | 0,10 | 0,10 |
| Parfüm | 0,12 | 0,12 | 0,12 | 0,12 |
| C11-13 Isoparaffin | 0,06 | 0,06 | 0,06 | 0,06 |
| Wasser | 86,75 | 86,34 | 85,94 | 85,50 |
| | | | | |
| Viskosität Färbecreme [mPas]* | 8722 | 12180 | 14335 | 12360 |
| Viskosität Anwendungsmischung [mPas]** | 6256 | 9611 | 11840 | 6726 |

| | | | | |
|---|---|---|---|---|
| *Haake VT550, 20°C, SV **Haake VT550, 20°C, MV II | | | | |

Um den Einfluss des Gehalts an Kationpolymer, speziell an Polyquaternium-22, zu untersuchen, wurden die in Tabelle 9 notierten vier Testzusammensetzungen hergestellt, und ihre Viskositäten wurden gemessen. Die vier Testzusammensetzungen wurden jeweils im Gewichtsverhältnis 1:1 mit der Oxidationsmittelzusammensetzung gemäß der vorstehenden Tabelle 2 zu Haar aufhellenden Anwendungsmischungen vermischt, deren Viskositäten ebenfalls gemessen wurden.

Die nicht erfindungsgemäße Creme V1 und ihre Anwendungsmischung mit der Oxidationsmittelzusammensetzung weisen Viskositäten auf, die für die Anwendung auf dem Haar etwas zu gering und daher nicht optimal ist.

Die nicht erfindungsgemäße Creme V2 selbst weist zwar eine anwendungstechnisch praktikable Viskosität auf. Ihre Anwendungsmischung mit der Oxidationsmittelzusammensetzung verliert demgegenüber aber deutlich an Viskosität.

Erst durch den Zusatz des Kationpolymers (E2, E3) werden Viskositäten erzielt, die auch nach dem Vermischen mit der Oxidationsmittelzusammensetzung weitgehend stabil bleiben und damit optimale Anwendungseigenschaften ermöglichen.

## Patentansprüche

1. Mittel zur oxidativen Haaraufhellung oder Haarfärbung, enthaltend, jeweils bezogen auf das Gewicht des Mittels,
- 70 - 90 Gew.-%, bevorzugt 73 - 86 Gew.-%, besonders bevorzugt 76 - 84 Gew.-%, Wasser,
- mindestens ein Alkalisierungsmittel,
- mindestens ein vernetztes Copolymer, aufgebaut aus Acrylsäure und nicht-ethoxylierten Estern der Acrylsäure mit linearen C10-C30-Monoalkoholen als Monomeren, wobei das vernetzte Copolymer in einer Gesamtmenge von 0,2 - 0,6 Gew.-%, bevorzugt 0,3 - 0,5 Gew.-%, besonders bevorzugt 0,4 - 0,45 Gew.-%, jeweils bezogen auf das Gewicht des Mittels, enthalten ist,
- mindestens ein lineares, gesättigtes 1-Alkanol mit einer Hydroxygruppe und 8 bis 22 Kohlenstoffatomen in einer Gesamtmenge von 0,3 - 1,5 Gew.-%, bevorzugt 0,4 - 1,2 Gew.-%, besonders bevorzugt 0,5 - 1,0 Gew.-%, außerordentlich bevorzugt 0,6 - 0,8 Gew.-%,
- mindestens ein anionisches Tensid in einer Gesamtmenge von 1 - 6 Gew.-%, bevorzugt 2 - 5 Gew.-%, und besonders bevorzugt 2,5 - 4,5 Gew.-%, und
- mindestens ein Polymer, ausgewählt aus kationischen und zwitterionischen Polymeren, in einer Gesamtmenge von 0,1 - 2 Gew.-%, bevorzugt 0,2 - 1,5 Gew.-%, besonders bevorzugt 0,35 - 0,5 Gew.-%,
- optional mindestens eine färbende Substanz, ausgewählt aus Oxidationsfarbstoffvorprodukten, direktziehenden Haarfarbstoffen und Mischungen hiervon,
wobei keine Oxidationsmittel enthalten sind,
**dadurch gekennzeichnet, dass**
- Natriumpolyacrylat, bevorzugt mit einer massenmittleren Molmasse M_{w} im Bereich von 1.000.000 bis 20.000.000 Dalton, besonders bevorzugt 6.000.000 bis 15.000.000 Dalton, enthalten ist, bevorzugt in einer Gesamtmenge von 0,1 - 1,5 Gew.-%, besonders bevorzugt 0,5 - 1,3 Gew.-%, außerordentlich bevorzugt 0,8 - 1,1 Gew.-%, jeweils bezogen auf das Gewicht des Mittels, wobei besonders bevorzugt das Natriumpolyacrylat als in einer Wasser-in-ÖI-Emulsion vorgeliert enthalten ist.

2. Mittel nach Anspruch 1, **dadurch gekennzeichnet, dass** das Alkalisierungsmittel ausgewählt ist aus der Gruppe, umfassend Ammoniumhydroxid, basische Aminosäuren, Alkalihydroxide, Alkanolamine, Alkalimetallmetasilikate, Alkaliphosphate und Alkalihydrogenphosphate sowie Mischungen hiervon, besonders bevorzugt ausgewählt ist aus der Gruppe, umfassend Alkanolamine, Kaliumhydroxid, Natriumhydroxid sowie Mischungen hiervon.

3. Mittel nach Anspruch 1 oder 2, **dadurch gekennzeichnet, dass** das mindestens eine anionische Tensid ausgewählt ist aus C₈-C₂₀-Alkylsulfaten, C₈-C₂₀-Alkylethersulfaten und C₈-C₂₀-Ethercarbonsäuren, jeweils mit 8 bis 20 C-Atomen in der Alkylgruppe und 0 bis 12 Ethylenoxidgruppen im Molekül, wobei bevorzugt Natriumlaurethsulfat mit 1 bis 3, besonders bevorzugt 2, Ethylenoxidgruppen im Molekül, enthalten ist.

4. Mittel nach einem der Ansprüche 1 - 3, **dadurch gekennzeichnet, dass** das mindestens eine vernetzte Copolymer aus Acrylsäure und nicht-ethoxylierten Estern der Acrylsäure mit linearen C10-C30-Monoalkoholen ausgewählt ist aus Copolymeren mit der INCI-Bezeichnung Acrylates/C10-30 Alkyl Acrylate Crosspolymer.

5. Mittel nach einem der Ansprüche 1 - 4, **gekennzeichnet durch** einen pH-Wert im Bereich von 7,5 - 12, bevorzugt 9 - 11, besonders bevorzugt 9,5 - 10,5, jeweils gemessen bei 20°C.

6. Mittel nach einem der Ansprüche 1 - 5, **gekennzeichnet durch** eine Viskosität im Bereich von 8000 - 40.000 mPas, bevorzugt 10.000 - 35.000 mPas, besonders bevorzugt 12.000 - 30.000 mPas, liegt, jeweils gemessen bei 20°C mit einem Rotationsviskosimeter Haake VT 550, Messsystem SV.

7. Mittel nach einem der Ansprüche 1 - 6, **dadurch gekennzeichnet, dass** mindestens ein nichtionisches Tensid enthalten ist, das bevorzugt ausgewählt ist aus mit 7 - 80 Mol Ethylenoxid pro Mol ethoxyliertem Rizinusöl, ethoxylierten C₈-C₃₀-Alkanolen mit 6 - 30, bevorzugt 12 bis 20 Mol Ethylenoxid pro Mol, ethoxylierten C₈-C₃₀-Carbonsäuren mit 5 - 30 Mol Ethylenoxid pro Mol, mit 4 - 50 Mol Ethylenoxid pro Mol ethoxylierten Sorbitanmonoestern von linearen gesättigten und ungesättigten C₁₂ - C₃₀-Carbonsäuren, die hydroxyliert sein können, Alkylmono- und -oligoglycosiden mit 8 bis 22 Kohlenstoffatomen im Alkylrest, sowie Mischungen der vorgenannten Substanzen.

8. Mittel nach einem der Ansprüche 1 - 7, **dadurch gekennzeichnet, dass** das mindestens eine kationische oder zwitterionische Polymer ausgewählt ist aus Polyquaternium-22, Polyquaternium-39, Acrylamidopropyltrimonium chloride/Acrylate Copolymer und Polyquaternium-6 sowie Mischungen hiervon, wobei Polyquaternium-22 besonders bevorzugt ist.

9. Mittel nach einem der Ansprüche 1 - 8, **dadurch gekennzeichnet, dass** mindestens ein Öl, bevorzugt in einer Gesamtmenge von 0,01 - 7 Gew.-%, weiter bevorzugt 0,1 - 6 Gew.-%, besonders bevorzugt 0,5- 5,5 Gew.-%, außerordentlich bevorzugt 1 - 5,2 Gew.-%, jeweils bezogen auf das Gewicht des Mittels, enthalten ist.

10. Mittel nach einem der Ansprüche 1 - 9, **dadurch gekennzeichnet, dass** das mindestens eine lineare, gesättigte 1-Alkanol mit einer Hydroxygruppe und 8 bis 22 Kohlenstoffatomen ausgewählt ist aus Laurylalkohol, Kokosalkohol,1-Tetradecanol (Myristylalkohol), Cetylalkohol und Stearylalkohol sowie Mischungen hiervon, insbesondere aus Kokosalkohol und Laurylalkohol.

11. Mittel nach einem der Ansprüche 1 - 10, **dadurch gekennzeichnet, dass** das mindestens eine nichtionische Tensid in einer Gesamtmenge von 0,01 - 1 Gew.-%, bevorzugt 0,05 - 0,5 Gew.-%, und besonders bevorzugt 0,1 - 0,3 Gew.-%, jeweils bezogen auf das Gewicht des Mittels, enthalten ist.

12. Mittel nach einem der Ansprüche 1 - 11, **dadurch gekennzeichnet, dass** das mindestens eine Öl ausgewählt ist aus natürlichen und synthetischen Kohlenwasserstoffen, besonders bevorzugt aus Mineralöl, Paraffinölen, C₁₈-C₃₀-Isoparaffinen, insbesondere Isoeicosan, Polyisobutenen und Polydecenen, C₈-C₁₆-Isoparaffinen, sowie 1,3-Di-(2-ethylhexyl)-cyclohexan; verzweigten Alkanolen mit einer Hydroxygruppe und 10 bis 50 Kohlenstoffatomen, den Benzoesäureestern von linearen oder verzweigten C₈₋₂₂-Alkanolen; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Atkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

13. Mittel nach einem der Ansprüche 1 - 12, **dadurch gekennzeichnet, dass** mindestens eine farbgebende Substanz, ausgewählt aus Oxidationsfarbstoffvorprodukten, bevorzugt in einer Menge von 0,005 - 5 Gew.-%, bezogen auf das Gewicht des Mittels, enthalten ist, wobei besonders bevorzugt mindestens eine Entwicklerkomponente und optional mindestens eine Kupplerkomponente enthalten ist, wobei außerordentlich bevorzugt die Gesamtmenge an Oxidationsfarbstoffvorprodukten 0,005 - 12 Gew.-%, bevorzugt 0,1 - 7 Gew.-%, besonders bevorzugt 0,5 - 5 Gew.-%, jeweils bezogen auf das Gewicht des Mittels, beträgt.

14. Verpackungseinheit (Kit-of-Parts), umfassend - getrennt voneinander verpackt -
a) mindestens einen Container (C1), enthaltend ein Mittel zur oxidativen Haaraufhellung oder Haarfärbung nach einem der Ansprüche 1 bis 13, und
b) mindestens einen Container (C2), enthaltend eine Oxidationsmittelzubereitung (M2), die 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-%, enthält und einen pH-Wert im Bereich von 2,0 bis 6,5, bevorzugt 2,5 - 5,5, besonders bevorzugt 2,8 bis 5,0, aufweist, jeweils gemessen bei 20°C, wobei sich die Gew.-%-Angaben jeweils auf das Gewicht der Oxidationsmittelzubereitung (M2) beziehen.

15. Verfahren zur oxidativen Haaraufhellung oder Haarfärbung, das die folgenden Verfahrensschritte umfasst:
i) Bereitstellen eines kosmetischen Mittels zur oxidativen Haarfärbung (M1) nach einem der Ansprüche 1 bis 13,
ii) Bereitstellen einer Oxidationsmittelzubereitung (M2), enthaltend 40 - 96 Gew.-%, bevorzugt 70 - 93 Gew.-%, besonders bevorzugt 80 - 90 Gew.-%, Wasser, weiterhin Wasserstoffperoxid in einer Gesamtmenge von 0,5 bis 23 Gew.-%, weiter bevorzugt 2,5 bis 13 Gew.-%, besonders bevorzugt 3 bis 10 Gew.-%, ganz besonders bevorzugt 6 bis 9 Gew.-%, und einen pH-Wert im Bereich von 2,0 bis 6,5, bevorzugt 2,5 - 5,5, besonders bevorzugt 2,8 bis 5,0, aufweisend, jeweils gemessen bei 20°C,
wobei optional mindestens ein Copolymer, ausgewählt aus vernetzten Acrylsäure/Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (M2), enthalten sein kann,
iii) Vermischen des kosmetischen Mittels (M1) mit der Oxidationsmittelzubereitung (M2), bevorzugt in einem Gewichtsverhältnis (M1):(M2) im Bereich von 0,5:1: bis 3:1, bevorzugt 0,8:1 bis 2,5:1 , besonders bevorzugt 1:1 bis 1,5:1, direkt anschließend
iv) Applizieren der in Schritt iii) erhaltenen Mischung auf die Haare und Belassen dieser Mischung für eine Zeit von 1 bis 60 Minuten, bevorzugt von 20 bis 45 Minuten, bei Raumtemperatur und/oder bei 30 - 60°C auf den Haaren,
v) Spülen der Haare mit Wasser und/oder einer reinigenden Zusammensetzung, und
vi) gegebenenfalls Applizieren eines Nachbehandlungsmittels auf die Haare und gegebenenfalls Ausspülen, anschließend Trocknen.

## Claims

1. An agent for oxidative hair lightening or hair coloring, containing, in each case based on the weight of the agent,
- 70-90 wt.%, preferably 73-86 wt.%, particularly preferably 76-84 wt.%, water,
- at least one alkalizing agent,
- at least one crosslinked copolymer made up of acrylic acid and non-ethoxylated esters of acrylic acid with linear C10-C30 monoalcohols as monomers, the crosslinked copolymer being contained in a total amount of 0.2-0.6 wt.%, preferably 0.3-0.5 wt.%, particularly preferably 0.4-0.45 wt.%, in each case based on the weight of the agent,
- at least one linear, saturated 1-alkanol having one hydroxy group and 8 to 22 carbon atoms in a total amount of 0.3-1.5 wt.%, preferably 0.4-1.2 wt.%, particularly preferably 0.5-1.0 wt.%, extremely preferably 0.6-0.8 wt.%,
- at least one anionic surfactant in a total amount of 1-6 wt.%, preferably 2-5 wt.%, and particularly preferably 2.5-4.5 wt.%, and
- at least one polymer selected from cationic and zwitterionic polymers in a total amount of 0.1-2 wt.%, preferably 0.2-1.5 wt.%, particularly preferably 0.35-0.5 wt.%,
- optionally at least one coloring substance selected from oxidation dye precursors, direct hair dyes and mixtures thereof,
no oxidizing agents being contained,
**characterized in that**
- sodium polyacrylate, preferably having a mass-average molar mass M_{W} in the range of 1,000,000 to 20,000,000 daltons, particularly preferably 6,000,000 to 15,000,000 daltons, is contained, preferably in a total amount of 0.1-1.5 wt.%, particularly preferably 0.5-1.3 wt.%, extremely preferably 0.8-1.1 wt.%, in each case based on the weight of the agent, the sodium polyacrylate particularly preferably being pregelled in a water-in-oil emulsion.

2. The agent according to claim 1, **characterized in that** the alkalizing agent is selected from the group comprising ammonium hydroxide, basic amino acids, alkali hydroxides, alkanolamines, alkali metal metasilicates, alkali phosphates, alkali hydrogen phosphates and mixtures thereof, said agent being particularly preferably selected from the group comprising alkanolamines, potassium hydroxide, sodium hydroxide and mixtures thereof.

3. The agent according to claim 1 or 2, **characterized in that** the at least one anionic surfactant is selected from C₈-C₂₀ alkyl sulfates, C₈-C₂₀ alkyl ether sulfates and C₈-C₂₀ ether carboxylic acids, each having 8 to 20 C atoms in the alkyl group and 0 to 12 ethylene oxide groups in the molecule, sodium laureth sulfate having 1 to 3, particularly preferably 2, ethylene oxide groups in the molecule being preferably contained.

4. The agent according to one of claims 1-3, **characterized in that** the at least one crosslinked copolymer of acrylic acid and non-ethoxylated esters of acrylic acid with linear C10-C30 monoalcohols is selected from copolymers with the INCI name acrylates/C10-30 alkyl acrylate crosspolymer.

5. The agent according to one of claims 1-4, **characterized by** a pH in the range of 7.5-12, preferably 9-11, particularly preferably 9.5-10.5, measured in each case at 20 °C.

6. The agent according to one of claims 1-5, **characterized by** a viscosity in the range of 8,000-40,000 mPas, preferably 10,000-35,000 mPas, particularly preferably 12,000-30,000 mPas, measured in each case at 20 °C using a Haake VT 550 rotational viscometer, measuring system SV.

7. The agent according to one of claims 1-6, **characterized in that** at least one non-ionic surfactant is contained, which is preferably selected from castor oil ethoxylated with 7-80 mol of ethylene oxide per mol, ethoxylated C₈-C₃₀ alkanols having 6-30, preferably 12-20, mol of ethylene oxide per mol, ethoxylated C₈-C₃₀ carboxylic acids having 5-30 mol of ethylene oxide per mol, sorbitan monoesters of linear saturated and unsaturated C₁₂-C₃₀ carboxylic acids which are ethoxylated with 4-50 mol of ethylene oxide per mol and may be hydroxylated, alkyl mono- and oligoglycosides having 8 to 22 carbon atoms in the alkyl group, and mixtures of the aforementioned substances.

8. The agent according to one of claims 1-7, **characterized in that** the at least one cationic or zwitterionic polymer is selected from polyquaternium-22, polyquaternium-39, acrylamidopropyltrimonium chloride/acrylate copolymer, polyquaternium-6 and mixtures thereof, polyquaternium 22 being particularly preferred.

9. The agent according to one of claims 1-8, **characterized in that** at least one oil is contained, preferably in a total amount of 0.01-7 wt.%, more preferably 0.1-6 wt.%, particularly preferably 0.5-5.5 wt.%, extremely preferably 1-5.2 wt.%, in each case based on the weight of the agent.

10. The agent according to one of claims 1-9, **characterized in that** the at least one linear, saturated 1-alkanol having one hydroxy group and 8 to 22 carbon atoms is selected from lauryl alcohol, coconut alcohol, 1-tetradecanol (myristyl alcohol), cetyl alcohol, stearyl alcohol and mixtures thereof, in particular from coconut alcohol and lauryl alcohol.

11. The agent according to one of claims 1-10, **characterized in that** the at least one non-ionic surfactant is contained in a total amount of 0.01-1 wt.%, preferably 0.05-0.5 wt.%, particularly preferably 0.1-0.3 wt.%, in each case based on the weight of the agent.

12. The agent according to one of claims 1-11, **characterized in that** the at least one oil is selected from natural and synthetic hydrocarbons, particularly preferably from mineral oil, paraffin oils, C₁₈-C₃₀ isoparaffins, in particular isoeicosane, polyisobutenes and polydecenes, C₈-C₁₆ isoparaffins, and 1,3-di-(2-ethylhexyl)cyclohexane; branched alkanols having one hydroxy group and 10 to 50 carbon atoms, the benzoic acid esters of linear or branched C₈₋₂₂ alkanols; triglycerides of linear or branched, saturated or unsaturated, optionally hydroxylated C₈₋₃₀ fatty acids, in particular natural oils; the dicarboxylic acid esters of linear or branched C₂-C₁₀ alkanols; esters of linear or branched, saturated or unsaturated fatty alcohols having 2-30 carbon atoms with linear or branched, saturated or unsaturated fatty acids having 2-30 carbon atoms, which may be hydroxylated; the addition products of 1 to 5 propylene oxide units to mono- or polyvalent C₈₋₂₂ alkanols; the C₈-C₂₂ fatty alcohol esters of monovalent or polyvalent C₂-C₇ hydroxycarboxylic acids; the symmetrical, unsymmetrical or cyclic esters of carbonic acid with C₃₋₂₂ alkanols, C₃₋₂₂ alkanediols or C₃₋₂₂ alkanetriols; esters of dimers of unsaturated C₁₂-C₂₂ fatty acids (dimer fatty acids) with monovalent linear, branched or cyclic C₂-C₁₈ alkanols or with polyvalent linear or branched C₂-C₆ alkanols; silicone oils and mixtures of the aforementioned substances.

13. The agent according to one of claims 1-12, **characterized in that** at least one color-imparting substance, selected from oxidation dye precursors, is contained, preferably in an amount of 0.005-5 wt.%, based on the weight of the agent, at least one developer component and optionally at least one coupler component being particularly preferably contained, the total amount of oxidation dye precursors being extremely preferably 0.005-12 wt.%, preferably 0.1-7 wt.%, particularly preferably 0.5-5 wt.%, in each case based on the weight of the agent.

14. A packaging unit (kit of parts) comprising, packaged separately from one another,
a) at least one container (C1) containing an agent for oxidative hair lightening or hair coloring according to one of claims 1 to 13, and
b) at least one container (C2) containing an oxidizing agent preparation (M2) which contains 40-96 wt.%, preferably 70-93 wt.%, particularly preferably 80-90 wt.%, water, and hydrogen peroxide in a total amount of 0.5 to 23 wt.%, more preferably 2.5 to 13 wt.%, particularly preferably 3 to 10 wt.%, very particularly preferably 6 to 9 wt.%, and has a pH in the range of 2.0 to 6.5, preferably 2.5 to 5.5, particularly preferably 2.8 to 5.0, measured in each case at 20 °C, the percentages by weight in each case relating to the weight of the oxidizing agent preparation (M2).

15. A method for oxidative hair lightening or hair coloring, comprising the following method steps:
i) providing a cosmetic agent for oxidative hair coloring (M1) according to one of claims 1 to 13,
ii) providing an oxidizing agent preparation (M2) which contains 40-96 wt.%, preferably 70-93 wt.%, particularly preferably 80-90 wt.%, water, and hydrogen peroxide in a total amount of 0.5 to 23 wt.%, more preferably 2.5 to 13 wt.%, particularly preferably 3 to 10 wt.%, very particularly preferably 6 to 9 wt.%, and has a pH in the range of 2.0 to 6.5, preferably 2.5 to 5.5, particularly preferably 2.8 to 5.0, measured in each case at 20 °C,
it being possible for at least one copolymer selected from crosslinked acrylic acid/acrylic acid C1-C6 alkyl ester copolymers and crosslinked methacrylic acid/acrylic acid C1-C6 alkyl ester copolymers to be optionally contained, preferably in a total amount of 0.1-7 wt.%, particularly preferably 0.5-6 wt.%, extremely preferably 1-4.5 wt.%, in each case based on the weight of the oxidizing agent preparation (M2),
iii) mixing the cosmetic agent (M1) with the oxidizing agent preparation (M2), preferably in a weight ratio (M1):(M2) in the range of 0.5:1 to 3:1, preferably 0.8:1 to 2.5:1, particularly preferably 1:1 to 1.5:1, and immediately afterwards
iv) applying the mixture obtained in step iii) to the hair and leaving said mixture on the hair for a time of from 1 to 60 minutes, preferably from 20 to 45 minutes, at room temperature and/or at 30-60 °C,
v) rinsing the hair with water and/or a cleansing composition, and
vi) optionally applying an aftertreatment agent to the hair and optionally rinsing said agent out, and then drying the hair.

## Revendications

1. Agent d'éclaircissement ou de coloration des cheveux par oxydation, contenant, respectivement par rapport au poids de l'agent,
- 70 à 90 % en poids, de préférence 73 à 86 % en poids, de manière particulièrement préférée 76 à 84 % en poids d'eau,
- au moins un agent d'alcalinisation,
- au moins un copolymère réticulé composé d'acide acrylique et d'esters non éthoxylés de l'acide acrylique comportant des monoalcools linéaires en C10-C30 comme monomères, le copolymère réticulé étant contenu en une quantité totale de 0,2 à 0,6 % en poids, de préférence de 0,3 à 0,5 % en poids, de manière particulièrement préférée de 0,4 à 0,45 % en poids, respectivement par rapport au poids de l'agent,
- au moins un 1-alcanol saturé linéaire comportant un groupe hydroxy et 8 à 22 atomes de carbone en une quantité totale de 0,3 à 1,5 % en poids, de préférence de 0,4 à 1,2 % en poids, de manière particulièrement préférée de 0,5 à 1,0 % en poids, de manière préférée entre toutes de 0,6 à 0,8 % en poids,
- au moins un tensioactif anionique en une quantité totale de 1 à 6 % en poids, de préférence de 2 à 5 % en poids, et de manière particulièrement préférée de 2,5 à 4,5 % en poids, et
- au moins un polymère choisi parmi les polymères cationiques et zwitterioniques en une quantité totale de 0,1 à 2 % en poids, de préférence de 0,2 à 1,5 % en poids, de manière particulièrement préférée de 0,35 à 0,5 % en poids,
- éventuellement au moins une substance colorante choisie parmi les précurseurs de colorants d'oxydation, les colorants capillaires à action directe et leurs mélanges,
dans lequel n'est contenu aucun agent d'oxydation,
**caractérisé en ce que**
- du polyacrylate de sodium, de préférence comportant une masse molaire moyenne en masse M_{W} dans la plage de 1 000 000 à 20 000 000 daltons, de manière particulièrement préférée de 6 000 000 à 15 000 000 daltons, est contenu, de préférence en une quantité totale de 0,1 à 1,5 % en poids, de manière particulièrement préférée de 0,5 à 1,3 % en poids, de manière préférée entre toutes de 0,8 à 1,1 % en poids, respectivement par rapport au poids de l'agent, le polyacrylate de sodium étant contenu de manière particulièrement préférée comme une émulsion eau-dans-huile prégélifiée.

2. Agent selon la revendication 1, **caractérisé en ce que** l'agent d'alcalinisation est choisi dans le groupe comprenant l'hydroxyde d'ammonium, les acides aminés basiques, les hydroxydes alcalins, les alcanolamines, les métasilicates de métaux alcalins, les phosphates alcalins et les hydrogénophosphates alcalins et leurs mélanges, choisi de manière particulièrement préférée dans le groupe comprenant les alcanolamines, l'hydroxyde de potassium, l'hydroxyde de sodium et leurs mélanges.

3. Agent selon la revendication 1 ou 2, **caractérisé en ce que** l'au moins un tensioactif anionique est choisi parmi les sulfates d'alkyle en C₈-C₂₀, les sulfates d'alkyléther en C₈-C₂₀ et les acides éthercarboxyliques en C₈-C₂₀, comportant respectivement 8 à 20 atomes de carbone dans le groupe alkyle et 0 à 12 groupes oxyde d'éthylène dans la molécule, le laurethsulfate de sodium comportant 1 à 3, de manière particulièrement préférée 2, groupes oxyde d'éthylène étant de préférence contenu dans la molécule.

4. Agent selon l'une des revendications 1 à 3, **caractérisé en ce que** l'au moins un copolymère réticulé composé d'acide acrylique et d'esters non éthoxylés de l'acide acrylique comportant des monoalcools linéaires en C10-C30 est choisi parmi les copolymères de désignation INCI acrylates/polymère croisé d'acrylate d'alkyle en C10-30.

5. Agent selon l'une des revendications 1 à 4, **caractérisé par** une valeur de pH dans la plage de 7,5 à 12, de préférence de 9 à 11, de manière particulièrement préférée de 9,5 à 10,5, respectivement mesurée à 20 °C.

6. Agent selon l'une des revendications 1 à 5, **caractérisé par** une viscosité dans la plage de 8 000 à 40 000 mPas, de préférence de 10 000 à 35 000 mPas, de manière particulièrement préférée de 12 000 à 30 000 mPas, respectivement mesurée à 20 °C avec un viscosimètre rotatif Haake VT 550, système de mesure SV.

7. Agent selon l'une des revendications 1 à 6, **caractérisé en ce qu'**au moins un tensioactif non ionique est contenu, qui est de préférence choisi parmi l'huile de ricin éthoxylée et comportant 7 à 80 moles d'oxyde d'éthylène par mole, les alcanols en Cs-C₃₀ éthoxylés et comportant 6 à 30, de préférence 12 à 20, moles d'oxyde d'éthylène par mole, les acides carboxyliques en C₈-C₃₀ éthoxylés et comportant 5 à 30 moles d'oxyde d'éthylène par mole, les monoesters de sorbitane éthoxylés et comportant 4 à 50 moles d'oxyde d'éthylène par mole d'acides carboxyliques linéaires saturés et insaturés en C₁₂-C₃₀, lesquels peuvent être hydroxylés, les monoglycosides et oligoglycosides d'alkyle comportant 8 à 22 atomes de carbone dans le radical alkyle, et les mélanges des substances susmentionnées.

8. Agent selon l'une des revendications 1 à 7, **caractérisé en ce que** l'au moins un polymère cationique ou zwitterionique est choisi parmi le polyquaternium-22, le polyquaternium-39, le copolymère de chlorure/d'acrylates d'acrylamidopropyltrimonium et le polyquaternium-6 et leurs mélanges, le polyquaternium-22 étant particulièrement préféré.

9. Agent selon l'une des revendications 1 à 8, **caractérisé en ce qu'**il contient au moins une huile, de préférence en une quantité totale de 0,01 à 7 % en poids, de manière davantage préférée de 0,1 à 6 % en poids, de manière particulièrement préférée de 0,5 à 5,5 % en poids, de manière préférée entre toutes de 1 à 5,2 % en poids, respectivement par rapport au poids de l'agent.

10. Agent selon l'une des revendications 1 à 9, **caractérisé en ce que** l'au moins un 1-alcanol saturé linéaire comportant un groupe hydroxy et de 8 à 22 atomes de carbone est choisi parmi l'alcool laurique, l'alcool de coco, le 1-tétradécanol (alcool myristylique), l'alcool cétylique et l'alcool stéarylique et leurs mélanges, en particulier parmi l'alcool de coco et l'alcool laurique.

11. Agent selon l'une des revendications 1 à 10, **caractérisé en ce que** l'au moins un tensioactif non ionique est contenu en une quantité totale de 0,01 à 1 % en poids, de préférence de 0,05 à 0,5 % en poids, et de manière particulièrement préférée de 0,1 à 0,3 % en poids, respectivement par rapport au poids de l'agent.

12. Agent selon l'une des revendications 1 à 11, **caractérisé en ce que** l'au moins une huile est choisie parmi les hydrocarbures naturels et synthétiques, de manière particulièrement préférée parmi l'huile minérale, les huiles de paraffine, les isoparaffines en C₁₈-C₃₀, en particulier l'isoéicosane, les polyisobutènes et les polydécènes, les isoparaffines en C₈-C₁₆, ainsi que le 1,3-di-(2-éthylhexyl)-cyclohexane ; les alcanols ramifiés comportant un groupe hydroxy et 10 à 50 atomes de carbone, les esters d'acide benzoïque d'alcanols en C₈₋₂₂ linéaires ou ramifiés ; les triglycérides d'acides gras en C₈₋₃₀ linéaires ou ramifiés, saturés ou insaturés, éventuellement hydroxylés, en particulier d'huiles naturelles ; les esters d'acide dicarboxylique d'alcanols en C₂-C₁₀ linéaires ou ramifiés ; les esters d'alcools gras linéaires ou ramifiés, saturés ou insaturés, comportant 2 à 30 atomes de carbone comportant des acides gras linéaires ou ramifiés, saturés ou insaturés, comportant 2 à 30 atomes de carbone, lesquels peuvent être hydroxylés ; les produits d'addition de 1 à 5 unités oxyde de propylène sur des alcanols en C₈₋₂₂ monovalents ou multivalents ; les esters d'alcools gras en C₈-C₂₂ d'acides hydroxycarboxyliques en C₂-C₇ monovalents ou multivalents ; les esters symétriques, asymétriques ou cycliques de l'acide carbonique comportant des alcanols en C3-22, des alcanediols en C3-22 ou des alcanetriols en C3-22 ; les esters de dimères d'acides gras en C₁₂-C₂₂ insaturés (acides gras dimères) comportant des alcanols en C₂-C₁₈ monovalents linéaires, ramifiés ou cycliques ou comportant des alcanols en C₂-C₆ multivalents linéaires ou ramifiés ; les huiles de silicone et les mélanges des substances susmentionnées.

13. Agent selon l'une des revendications 1 à 12, **caractérisé en ce qu'**au moins une substance colorante, choisie parmi les précurseurs de colorants d'oxydation, est de préférence contenue en une quantité de 0,005 à 5 % en poids, par rapport au poids de l'agent, au moins un composant révélateur étant contenu de manière particulièrement préférée et éventuellement au moins un composant coupleur, la quantité totale de précurseurs de colorants d'oxydation étant de manière préférée entre toutes de 0,005 à 12 % en poids, de préférence de 0,1 à 7 % en poids, de manière particulièrement préférée de 0,5 à 5 % en poids, respectivement par rapport au poids de l'agent.

14. Unité d'emballage (kit de pièces) comprenant, emballés séparément les uns des autres,
a) au moins un récipient (C1) contenant un agent d'éclaircissement ou de coloration des cheveux par oxydation selon l'une des revendications 1 à 13, et
b) au moins un récipient (C2) contenant une préparation d'agent d'oxydation (M2) qui contient 40 à 96 % en poids, de préférence 70 à 93 % en poids, de manière particulièrement préférée 80 à 90 % en poids d'eau, ainsi que du peroxyde d'hydrogène en une quantité totale de 0,5 à 23 % en poids, de manière davantage préférée de 2,5 à 13 % en poids, de manière particulièrement préférée de 3 à 10 % en poids, de manière tout particulièrement préférée de 6 à 9 % en poids, et qui présente une valeur de pH dans la plage de 2,0 à 6,5, de préférence de 2,5 à 5,5, de manière particulièrement préférée de 2,8 à 5,0, respectivement mesurée à 20 °C, les pourcentages en poids se rapportant respectivement au poids de la préparation d'agent d'oxydation (M2).

15. Procédé d'éclaircissement ou de coloration des cheveux par oxydation, comprenant les étapes de procédé suivantes :
i) mise à disposition d'un agent cosmétique permettant la coloration par oxydation des cheveux (M1) selon l'une des revendications 1 à 13,
ii) fourniture d'au moins une préparation d'agent d'oxydation (M2) contenant 40 à 96 % en poids, de préférence 70 à 93 % en poids, de manière particulièrement préférée 80 à 90 % en poids d'eau, ainsi que du peroxyde d'hydrogène en une quantité totale de 0,5 à 23 % en poids, de manière davantage préférée de 2,5 à 13 % en poids, de manière particulièrement préférée de 3 à 10 % en poids, de manière tout particulièrement préférée de 6 à 9 % en poids, et présentant une valeur de pH dans la plage de 2,0 à 6,5, de préférence de 2,5 à 5,5, de manière particulièrement préférée de 2,8 à 5,0, respectivement mesurée à 20 °C, éventuellement au moins un copolymère choisi parmi les copolymères réticulés d'acide acrylique/d'acide acrylique alkylester en C1-C6 et les copolymères réticulés d'acide méthacrylique/d'acide acrylique alkylester en C1-C6, de préférence en une quantité totale de 0,1 à 7 % en poids, de manière particulièrement préférée de 0,5 à 6 % en poids, de manière préférée entre toutes de 1 à 4,5 % en poids, respectivement par rapport au poids de la préparation d'agent d'oxydation (M2), pouvant être contenu,
iii) mélange de l'agent cosmétique (M1) avec la préparation d'agent d'oxydation (M2), de préférence dans un rapport pondéral (M1):(M2) dans la plage de 0,5:1 à 3:1, de préférence de 0,8:1 à 2,5:1, de manière particulièrement préférée de 1:1 à 1,5:1, et ensuite
iv) application du mélange obtenu à l'étape iii) sur les cheveux et pose dudit mélange sur les cheveux pour une durée de 1 à 60 minutes, de préférence de 20 à 45 minutes, à température ambiante et/ou à une température de 30 à 60 °C,
v) rinçage des cheveux avec de l'eau et/ou une composition nettoyante, et
vi) application éventuelle d'un agent de post-traitement sur les cheveux et rinçage éventuel, puis séchage.
